# EUROPÄISCHE PATENTSCHRIFT

(11) **EP 3 660 087 B1**
(45) Veröffentlichungstag und Bekanntmachung des Hinweises auf die Patenterteilung: **29.05.2024**
(21) Anmeldenummer: 18209277.5
(22) Anmeldetag: 29.11.2018
(51) Int. Cl.: C08K 5/00

(54) **VERFAHREN UND WERKSTOFF ZUR HERSTELLUNG VON 3D OBJEKTEN DURCH ENERGIEIMPULS-INDUZIERTEN TRANSFERDRUCK**
METHOD AND MATERIAL FOR THE PRODUCTION OF THREE-DIMENSIONAL OBJECTS BY ENERGY IMPULSE-INDUCED TRANSFER PRINTING
PROCÉDÉ ET MATÉRIAU DESTINÉS À LA FABRICATION D'OBJETS TRIDIMENSIONNELS PAR IMPRESSION PAR TRANSFERT INDUIT PAR IMPULSION ÉNERGÉTIQUE

(43) Veröffentlichungstag der Anmeldung: 03.06.2020
(73) Patentinhaber: Ivoclar Vivadent AG, 9494 Schaan (LI); IO Tech Group, Ltd., London W10 6AZ (GB)
(72) Erfinder: BONDERER, Lorenz Josef, 7320 Sargans (CH); LAUBERSHEIMER, Jürgen Rudolf, 9470 Buchs (CH); WACHTER, Wolfgang Josef, 9494 Schaan (LI); ZENOU, Michael, 7312700 Hasmonaim (IL)
(74) Vertreter: Uexküll & Stolberg

(56) Entgegenhaltungen:
- EP-A1- 3 335 687
- DATABASE WPI Week 200838 Thomson Scientific, London, GB; AN 2008-F96087 XP002791806, -& JP 2008 094732 A (TOKUYAMA DENTAL KK) 24. April 2008 (2008-04-24)

## Beschreibung

Die Erfindung betrifft Verfahren zur Herstellung dreidimensionaler Objekte durch Energieimpuls-induzierten Transferdruck und die Verwendung von Werkstoffen als Baumaterialien in dem Verfahren. Die Materialien und Verfahren eigenen sich besonders zur Herstellung von Dentalrestaurationen.

Unter dem Begriff Additive Manufacturing AM werden generative Fertigungsverfahren zusammengefasst, bei denen aus computergestützten Konstruktionsdaten (CAD-Daten) dreidimensionale Modelle oder Bauteile hergestellt werden. Bekannte AM-Verfahren sind z.B. Stereolithographie (SL), selektives Lasersintern (SLS), 3D-Drucken, Fused Deposition Modelling (FDM), Inkjet-Printing (IJP), 3D-Plotting, Multi-Jet Modelling (MJM), Solid Free Form Fabrication (SFF), Laminated Object Manufacturing (LOM), Laser Powder Forming (LPF) und Direct Ceramic Jet-Printing (DCJP). Mit diesen Verfahren lassen sich Modelle, Bau- und Formteile auch in Kleinserie kostengünstig herstellen.

Die EP 1 268 211 B1 offenbart ein Druckverfahren, bei dem durch einen fokussierten Laserstrahl lokal eine Volumen- oder Positionsänderung in dem zu verdruckenden Material induziert wird, so dass sich ein Farbtröpfchen von der im wesentlichen homogenen Farbschicht ablöst und auf den Bedruckstoff übertragen wird. Dieses Verfahren wird als Laser Induced Forward Transfer (LIFT) Verfahren bezeichnet. Das zu verdruckende Material wird von dem sogenannten Donor oder Trägersubstrat auf das Empfängersubstrat (Akzeptor) übertragen. Das Trägersubstrat besteht aus einem Träger, der mit einer dünnen Schicht des zu verdruckenden Materials beschichtet ist. Diese Materialschicht wird mit einem Laser punktförmig bestrahlt und dabei erweicht oder geschmolzen und teilweise verdampft. Bei transparenten Trägern kann der Laser von der Rückseite durch den Träger hindurch auf das zu verdruckende Material fokussiert werden. Ist der Träger nicht transparent, wird der Träger durch den Laser erhitzt und das Material indirekt erweicht oder geschmolzen. Alternativ kann der Laser von oben direkt auf das Material gerichtet werden. Das Empfängersubstrat (Bedruckstoff) ist in einem geringen, genau einzuhaltenden Abstand zum Trägersubstrat angeordnet. Durch die Laserenergie wird ein Teil des zu verdruckenden Materials schlagartig verdampft. Die sich bildende Dampfwolke reisst eine kleine Menge des erweichten oder geschmolzenen Materials mit und scheidet es auf dem Empfängersubstrat ab.

Um das zu verdruckende Material zu verdampfen, muss das Laser-licht absorbiert und in Wärme umgewandelt werden. Bei Druckfarben wird der Laserstrahl in der Regel durch Farbpigmente absorbiert, die in den Farben enthalten sind. Alternativ kann eine Absorptionsschicht vorgesehen sein, die das Laserlicht absorbiert und die Energie dann an das zu verdruckende Material überträgt. Eine solche Absorptionsschicht ist üblicherweise zwischen dem Träger und dem zu verdruckenden Material angeordnet. Absorptionsschichten sind nachteilig, weil häufig Teile dieser Schicht zusammen mit der Druckfarbe auf das Empfängersubstrat übertragen werden.

Zenou et al., small 2015, 11, Nr. 33, 4082-4089 beschreiben die Herstellung dreidimensionaler Metallobjekte durch das LIFT-Verfahren. Hierzu setzen sie metallbeschichtete Glasplatten als Trägersubstrat ein. Das Metall wird durch einen Laser geschmolzen und tröpfchenweise auf das Empfängersubstrat übertragen. Durch das Drucken vieler Schichten übereinander werden dreidimensionale Metallstrukturen erhalten.

Beim 3D Druck mittels Stereolithographie müssen die verdruckbaren Materialien bei der Verarbeitung fliessfähig und photoreaktiv sein. Beim 3D Inkjet Printing liegt die Viskosität der Tinten deutlich unter 1 Pa·s und meist unter 0,1 Pa·s. Die Anforderungen an die Viskosität schließen höherviskose Materialen und Suspensionen mit einem hohen Füllstoffgehalt aus. Andererseits sind Werkstoffe mit hoher Viskosität und hohem Füllstoffgehalt bevorzugt, weil sich eine hohe Viskosität der Ausgangsstoffe und ein hoher Füllgrad positiv auf die physikalischen Eigenschaften der Werkstücke auswirken und die Herstellung von Objekten mit hoher Zähigkeit, hoher Bruchfestigkeit, guter Abrasionsbeständigkeit und einem hohen E-Modul ermöglichen.

Der Erfindung liegt die Aufgabe zugrunde, ein generatives Verfahren zur Herstellung dreidimensionaler Objekte Verfügung zu stellen, durch welches auch hochviskose Materialien verarbeitet werden können. Zudem sollen unterschiedliche Materialien gemeinsam zu einem Objekt verarbeitet werden können. Außerdem sollen Werkstoffe zur Verfügung gestellt werden, die sich zur Verwendung als Baumaterialien in diesem Verfahren eignen, insbesondere zur Herstellung von Dentalrestaurationen.

Diese Aufgabe wird durch ein generatives Verfahren zur Herstellung dreidimensionaler Objekte gelöst, das die folgenden Schritte umfasst:
(1) Flächiges Aufbringen eines Stütz-/Baumaterials auf einen Träger in definierter Schichtdicke, vorzugsweise in einer Schichtdicke von 3 - 300 µm, besonders bevorzugt 10 - 100 µm,
(2) Transfer eines Anteils des Stütz-/Baumaterials vom Trägersubstrat (Donor) auf ein Empfängersubstrat (Akzeptor) durch den lokalen, ortsselektiven Eintrag eines Energieimpulses, vorzugsweise eines Laserimpulses,
(3) Verfestigen des Stütz-/Baumaterials auf dem Empfängersubstrat, vorzugsweise durch Trocknen, Strahlenhärtung oder Ändern des Aggregatszustandes (z.B. durch Temperaturänderung),
(4) Wiederholen der Schritte (1)-(3) bis das gewünschte Objekt erstellt ist,
(5) ggf. Entfernen des Stützmaterials und optionales Reinigen des Objekts,
(6) optionales Nachvergüten des Objekts durch weiteres Härten, vorzugsweise durch Trocknung, Strahlung, Wärme oder eine Kombination davon,
(7) optionale mechanische Bearbeitung des Objekts, z.B. durch Trowalisieren oder manuelles Bearbeiten wie Schleifen oder Polieren.

Das Verfahren ist dadruch gekennzeichnet, dass als Baumaterial ein Werkstoff mit der in Anspruch 1 definierten Zusammensetzung verwendet wird.

Das Härten der Schicht in Schritt (3) muss nicht unmittelbar nach dem Aufbringen der einzelnen Schicht vorgenommen werden. Es ist auch möglich, zunächst mehrere Schichten nacheinander aufzubringen (Schritt 2) und diese Schichten dann gemeinsam zu härten. Die Gesamtdicke der gemeinsam zu härtenden Schichten sollte bevorzugt 30 bis 200 µm betragen. Die Schritte (2) und ggf. (1) werden daher vorzugsweise so oft wiederholt, bis die Gesamtdicke in diesem Bereich liegt.

In Schritt (1) wird das Stütz-/Baumaterial auf einen Träger aufgebracht und in Schritt (2) von dem Träger auf das Empfängersubstrat übertragen. Ein in Schritt (1) mit Stütz-/Baumaterial beschichteter Träger kann mehrfach zur Übertragung von Stütz-/Baumaterial auf das Empfängersubstrat verwendet werden, beispielsweise indem ein noch nicht verwendeter Bereich des beschichteten Trägers gewählt wird. Schritt (2) kann daher mehrfach wiederholt werden, ohne dass eine Wiederholung des Schritts (1) erforderlich ist.

Die Schritte (1) bis (3) werden so oft wiederholt, bis das gewünschte Objekt fertiggestellt ist. Gemäß einer bevorzugten Ausführungsform wird das Stütz-/Baumaterial im Anschluss an Schritt (3) geglättet, vorzugsweise mit einer Walze, Klinge, Fräse und/oder einem Abstreifer.

Da Sauerstoff aus der Luft die Polymerisation an der Oberfläche des Bauteils inhibieren kann, wird eine Härtung der Werkstoffe durch Polymerisation vorzugsweise unter Ausschluss von Luft (Sauerstoff) durchgeführt. Das Verfahren kann unter Schutzgas durchgeführt werden, beispielweise unter einer Glocke, die mit Schutzgas gespült wird. Als Schutzgas eignen sich inerte Gase wie z.B. N₂, CO₂ oder Edelgase wie z.B. He oder Ar. Gemäß einer bevorzugten Ausführungsform wird durch das Verdampfen der Volumenausdehnungskomponente in der Region oberhalb des Bauteils eine sauerstoffarme Atmosphäre erzeugt, welche ebenfalls einer Inhibierung entgegen wirkt.

Nach der Fertigstellung des Objekts wird dieses vorzugsweise in einem zusätzlichen Schritt (6) einer weiteren Härtung unterworfen, um restliche Monomere, die bei schichtweisen Härten zurückgeblieben sind, zu Härten. Hierzu wird der Körper in Abhängigkeit vom gewählten Initiator z.B. auf ca. 60°C erhitzt oder vorzugsweise z.B. für 1 bis 12 Minuten mit Licht bestrahlt.

Der als Baumaterial verwendete radikalisch polymerisierbare Werkstoff enthält:
(a) mindestens ein mono- oder multifunktionelles (Meth)acrylat oder eine Mischung davon als polymerisierbares Bindemittel,
(b) mindestens ein Mono(meth)acrylat als Volumenausdehnungskomponente,
(c) mindestens einen Photoinitiator für die radikalische Polymerisation und
(d) mindestens einen anorganischen oder organischen Farbstoff und/oder mindestens ein anorganisches oder organisches Pigment als Energietransformationskomponente.

In dem Verfahren können ein oder mehrere unterschiedliche Baumaterialien eingesetzt werden.

Das oder die Baumaterialien können zusammen mit einem Stützmaterial verarbeitete werden. Unter Stützmaterialien werden Materialen verstanden, die aus dem fertigen Objekt entfernt werden. Baumaterialien sind demgegenüber Materialien, die das Objekt bilden und die nach dem Entfernen des Stützmaterials zurück bleiben. Der Begriff "Stütz-/Baumaterial" ist so zu verstehen, dass entweder ein Stützmaterial oder ein Baumaterial verdruckt wird oder dass beide Materialien zusammen verwendet werden. Stütz- und Baumaterialen gemeinsam werden hierin auch als Druckmaterialien bezeichnet. Das Stützmaterial wird ggf. in Schritt (5) aus dem fertigen Objekt entfernt.

Bevorzugte Träger in **Schritt (1)** sind Polymerfolien, bevorzugt mit einer Dicke von 10 - 200 µm, insbesondere PET-, Polyimid-, und Polyvinylchlorid (PVC)-folien; Glasträger, vorzugsweise aus Floatglas oder Borosilikatglas; Träger aus nicht-metallischen, anorganischen, porösen oder nicht-porösen Werkstoffen; metallische Träger, vorzugsweise aus Edelstahl, Aluminium, Titanlegierungen, Kupferlegierungen wie Bronze oder Messing; Träger aus nicht-metallischen, anorganischen Werkstoffen wie keramische Träger, vorzugsweise aus ZrO₂, Al₂O₃, Zirconia toughened Alumina (ZTA), Alumina toughened Zirconia (ATZ), SiCₓ, SiNₓ, Diamond like carbon, Glassy Carbon, BN, B₄C oder AlN; oder Träger aus einer Kombinationen dieser Materialien. Die Träger werden so gewählt, dass sie sich gegenüber dem Stütz-/Baumaterial ausreichend inert verhalten, d.h. insbesondere nicht innerhalb der Anwendungszeit merklich vom Stütz- oder Baumaterial gequollen oder angegriffen werden.

Der Träger kann als Platte, Einwegband, Endlosband, Zylinder oder Hohlzylinder vorliegen. Die Arbeitsfläche kann eben oder gewölbt sein. Gewölbte Flächen sind bevorzugt um eine Achse gewölbt, wie z.B. die Mantelfläche eines Zylinders. Der beschichtete Träger wird hierin auch als Trägersubstrat bezeichnet.

Um die Bildung einer homogenen Schicht des Stütz- oder Baumaterials zu unterstützen, werden Stütz-/Baumaterial und Träger vorzugsweise aufeinander abgestimmt. Es wird eine geringe Grenzflächenspannung zwischen Stütz-/Baumaterial und Träger angestrebt. Für hydrophile Stütz-/Baumaterialen werden vorzugsweise hydrophile Träger- und/oder Empfängersubstrate verwendet, beispielsweise Glasträger, Cellophan oder hydrophile PET-Folien.

Oberflächen können z.B. durch Flamm-, Plasma- oder Ätzbehandlungen hydrophilisiert werden. Generell soll das Druckmaterial, d.h. Stütz- und/oder Baumaterial, den Träger gut benetzten. Durch die Zugabe eines Tensids zum Druckmaterial kann die Benetzung ebenfalls verbessert werden. Bei hydrophoben Stütz-/Baumaterialen sind hydrophobe Träger bevorzugt.

Das Stütz-/Baumaterial kann auf bekannte Weise auf den Träger aufgebracht werden, vorzugsweise durch Rakel- oder Doktorbladesysteme, mit Schlitzdüsen (mit oder ohne Dispenser), durch Flexo- oder Gravurdruck, Siebdruck, Tampondruck, Sprühbeschichtung oder durch eine Kombination dieser Verfahren. Generell sind hierzu alle im Stand der Technik bekannten Druckmethoden geeignet. Der beschichtete Träger wird hierin auch als Trägersubstrat bezeichnet.

Im Fall von Druckzylindern wird das Stütz-/Baumaterial vorzugweise kontinuierlich auf einen rotierenden Zylinder aufgetragen. Durch die Rotation wird die auf dem Zylinder gebildete Schicht des Materials in Richtung der Energiequelle, z.B. des Lasers, transportiert und dort verdruckt. Anschließend wird das verdruckte Material bei weiterer Rotation wieder ergänzt.

Trägerfolien können ebenfalls in kontinuierlichen Verfahren eingesetzt werden, beispielsweise indem sie als umlaufendes Band ausgebildet werden. Die beschichteten Folien können aber auch für die einmalige Verwendung konfektioniert sein.

In **Schritt (2)** wird ein Teil der eingebrachten Energie durch das Stütz-/Baumaterial absorbiert und in Wärme umgewandelt. Die Absorption findet vorzugsweise ohne eine zusätzliche Absorptionsschicht auf dem Trägersubstrat im Stütz-/Baumaterial selbst statt, so dass die mit derartigen Absorptionsschichten verbundenen Nachteile vermieden werden.

Die Energieabsorption bewirkt eine lokale, schlagartige Volumenausdehnung, beispielsweise eine Verdampfung, der Volumenausdehnungskomponente im Material und führt zum Ablösen des Stütz-/Baumaterials vom Trägersubstrat und zum Transfer auf das Empfängersubstrat. Dabei werden Tropfen des Stütz-/Baumaterials auf das Empfängersubstrat übertragen, wo sie koaleszieren können und beispielsweise einen homogenen Film bilden.

Der Energieeintrag in Schritt (2) erfolgt vorzugsweise über die dem Stütz-/Baumaterial abgewandte Seite des Trägersubstrats.

Das Empfängersubstrat kann eine plane Oberfläche aufweisen und sollte mindestens so groß sein, dass es das zu druckende Bauteil in Gänze aufnehmen kann. Das Empfängersubstrat hat vorzugsweise eine glatte Oberfläche, die geschlossen oder porös sein kann. Unter einer porösen Oberfläche wird eine Oberfläche verstanden, die Poren mit einer mittleren Größe von vorzugsweise 1 nm - bis 10 µm aufweist. Die Porengröße wird rasterelektronenmikroskopisch durch Auszählen bestimmt. Angegeben sind die dabei erhaltenen Mittelwerte.

Beispielhafte Materialien mit mikro- oder nanoporöser Oberfläche sind abgebundener, trockener Gips, angesintertes aber noch poröses ZrO₂, nanoporöses Glas oder mikroporöse Kunststoffe, wie z.B. zusammengesintertes Polyethylen hoher Dichte.

Durch die Verwendung von porösen Empfängersubstraten kann das Trocknen der Stütz- und Baumaterialien begünstigt werden, besonders von solchen Baumaterialien, die feste Partikel enthalten, wie Schlicker für die Herstellung von keramischen Objekten. Besonders dann, wenn das Verfestigen durch Trocknen stattfindet, kann ein separater Trocknungsschritt entfallen. Es ist jedoch darauf zu achten, dass die Poren kleiner als die festen Füllstoffpartikel sind, damit diese nicht beim Trocknen die Poren verstopfen.

Erfindungsgemäß werden vorzugsweise Empfängersubstrate mit einer nicht porösen, d.h. geschlossenen, Oberfläche verwendet.

Die gewünschten dreidimensionalen Objekte werden durch wiederholtes schichtweises Verdrucken von Stütz- und Baumaterial hergestellt. Die einzelnen Schichten können jeweils allein durch das Stützmaterial, allein durch das Baumaterial oder durch beide Materialien gemeinsam gebildet werden.

Stütz- und Baumaterial können in einem Arbeitsgang gemeinsam oder nacheinander verdruckt werden. Beispielswese kann in einem ersten Arbeitsgang ein Stützmaterial verdruckt und dann in oder auf die verfestigte Stützstruktur auf die beschriebene Weise das Baumaterial gedruckt werden. Dabei können die abgeschiedenen Schichtstärken des Stützmaterials und des Baumaterials unterschiedlich sein. Dadurch kann es z.B. notwendig werden, dass die Anzahl abgeschiedener Schichten für Stütz- und Baumaterial unterschiedlich sind. Gemäß einer bevorzugten Ausführungsform werden zuerst mehrere Schichten mindestens eines Stützmaterials auf dem Empfängersubstrat abgeschieden.

Anschließend wird das gewünschte Objekt durch Verdrucken von mindestens einem Baumaterial gebildet. Nach der Fertigstellung des eigentlichen Bauteils können weitere Schichten des Stützmaterials aufgebracht werden, so das Ober- und Unterseite des gedruckten Objekts durch ein oder mehrere Schichten des Stützmaterials begrenzt sind. In einer besonders bevorzugten Ausführungsform wird in jeder Schicht der äußere Rand um das Bauobjekt herum durch das Stützmaterial gebildet, so dass das gedruckte Objekt allseitig von Stützmaterial umschlossen ist. In Bereichen des Bauteils, in denen sich der Querschnitt nicht stark verändert, können dickere Schichten verwendet werden, während bei Stellen, in denen sich der Bauteilquerschnitt schnell verändert, dünnere schichten bevorzugt sind.

Optional kann die aufgebrachte Materialschicht zur Vorbereitung für den nächsten Auftragungszyklus in einem weiteren Verfahrensschritt geglättet werden, beispielsweise mit einer Metallwalze, einer Klinge, einem Abstreifer oder einer Fräse mit/ohne Materialabsaugung.

Das schichtweise Aufbringen wird so lange fortgesetzt, bis das gewünschte dreidimensionale Objekt fertiggestellt ist. Der Druckvorgang wird wie bei generativen Fertigungsverfahren üblich durch einen Computer mittel CAD-Daten gesteuert. Dabei wird Baumaterial in den Bereichen verwendet, die das Formteil bilden, und das Stützmaterial unterhalb von Überhängen, seitlich des Bauteils und in Hohlräumen.

In einer bevorzugten Ausführungsform des Verfahrens wird das Druckmaterial, d.h. Stütz- oder Baumaterial, während des Druckprozesses auf den Träger aufgebracht. Alternativ können auch bereits vorgängig beschichtete Substrate verwendet werden, bevorzugt in Form von beschichteten Trägerfolien. Vorzugsweise wird durch erneutes, selektives oder kontinuierliches Beschichten des Trägersubstrats neues Druckmaterial für den LIFT-Vorgang zur Verfügung gestellt. Alternativ können auch bereits vorgängig beschichtete Substrate verwendet werden, bevorzugt in der Form von beschichteten Trägerfolien.

Falls ein Stützmaterial eingesetzt wird, muss dieses nach der Härtung des Werkstücks rückstandsfrei aus dem Formkörper entfernt werden, ohne dabei den Formkörper zu beschädigen **(Schritt 5),** beispielsweise durch Lösen in einem geeigneten Lösungsmittel und/oder Schmelzen. Außerdem kann das Stützmaterial auf maschinelle Weise entfernt werden, z.B. durch Trowalisieren, auf manuelle Weise, durch Ultraschallreinigung oder durch Abspritzen/Abwaschen. Kombinationen der genannten Maßnahmen kommen ebenfalls in Betracht. Beispielsweise kann das Stützmaterial durch Quellen in Lösungsmittel erweicht und dann maschinell oder manuell mechanisch entfernt werden. In einer besonders bevorzugten Ausführungsform wird das Stützmaterial geschmolzen und anhaftende Stützmaterialreste dann durch ein Lösungsmittelbad entfernt. Durch die beschriebenen Maßnahmen wird das Objekt gleichzeitig gereinigt.

Zur Nachvergütung **(Schritt 6)** kann das Bauteil vor, während oder bevorzugt nach der Reinigung durch Bestrahlung mit einer Strahlungsquelle, z.B. einer Quecksilberdampflampe oder LED-Lampe, nachgehärtet werden. Dieser Prozess kann durch Erwärmung des Bauteils bis maximal 120°C unterstützt werden.

Das erfindungsgemäße Verfahren ist ein LIFT-Verfahren. Unter einem LIFT-Verfahren wird hier ein Verfahren verstanden, bei dem wie eingangs erläutert durch einen Energieimpuls aus einem Druckmaterial eine kleine Materialmenge herausgelöst und auf ein Empfängersubstrat übertragen wird. Der Energieimpuls wird vorzugsweise durch einen Laser erzeugt. Der Laserstrahl wird dabei auf einen kleinen Bereich des Stütz- oder Baumaterials fokussiert und hierdurch das Stütz- oder Baumaterial lokal so stark erhitzt, dass sich die Volumenausdehnungskomponente schlagartig ausdehnt, z.B. durch Verdampfen eines Anteils des Druckmaterials. Die Energietransformationskomponente absorbiert die Laserenergie und überträgt diese auf die Volumenausdehnungskomponente. Die schlagartig verdampfende Volumenausdehnungskomponente reißt das Stütz- oder Baumaterial mit und überträgt es auf das Empfängersubstrat. Es ist auch möglich, dass die Volumenausdehnungskomponente einen Teil der Energie direkt absorbiert.

Erfindungsgemäß kann anstelle eines Laserstrahls eine andere geeignete Energiequelle verwendet werden, beispielsweise fokussiertes Licht (nicht Laser) oder Teilchenstrahlen wie Elektronen- oder Ionenstrahlen. Der Einfachheit halber wird hier der Begriff LIFT-Verfahren auch für Verfahren verwendet, in denen kein Laser eingesetzt wird. Bevorzugt sind Laser, insbesondere Laser mit einer Wellenlänge von 300 nm bis 4000 nm, beispielsweise Neodym-YAG Laser mit einer Wellenläge von 1064 nm. Besonders bevorzugt ist gepulstes Laser-Licht mit einer Pulsenergie im µJ Bereich und einer Pulsdauer von 1 ns bis 1 µs.

Das erfindungsgemäße Verfahren zeichnet sich dadurch aus, dass als Baumaterial ein Werkstoff verwendet wird, der als **Bindemittel (a)** mindestens ein mono- oder multifunktionelles (Meth)acrylat oder eine Mischung davon enthält. Unter monofunktionellen (Meth)acrylaten werden Verbindungen mit einer, unter mehrfach funktionellen (Meth)acrylaten Verbindungen mit zwei oder mehr, vorzugsweise 2 bis 3 polymerisierbaren Gruppen verstanden. Geeignete Beispiele sind Methyl-, Ethyl-, 2-Hydroxyethyl-, Butyl-, Benzyl-, Tetrahydrofurfuryl- oder Iso-bornyl(meth)acrylat, p-Cumylphenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertes Bisphenol-A-Dimethacrylat wie z.B. das Bisphenol-A-Dimethacrylat mit 3 Ethoxygruppen (SR-348c, Fa. Sartomer) oder 2,2-Bis[4-(2-methacryloxypropoxy)phenyl]propan, UDMA (ein Additionsprodukt aus 2-Hydroxyethylmethacrylat und 2,2,4-Trimethylhexamethylendiisocyanat), Di-, Tri- oder Tetraethylenglycoldi-(meth)acrylat, Trimethylolpropantri(meth)acrylat, Pentaery-thrittetra(meth)acrylat, sowie Glycerindi- und trimethacrylat, 1,4-Butandioldi(meth)acrylat, 1,10-Decandioldi(meth)acrylat (D3MA) oder 1,12-Dodecandioldi(meth)acrylat. Bevorzugte (Meth)acrylatmonomere sind Benzyl-, Tetrahydrofurfuryl- oder Isobornylmethacrylat, p-Cumyl-phenoxyethylenglycolmethacrylat, 2,2-Bis[4-(2-methacryloxypropoxy)phenyl]propan, Bis-GMA, UDMA, SR-348c und D3MA.

Vorzugsweise werden Mischungen der vorstehend genannten Monomere verwendet.

Die **Volumenausdehnungskomponente (b)** hat den Hauptzweck, einen Transfer des Stütz- oder Baumaterials vom Trägersubstrat auf das Empfängersubstrat zu bewirken. Damit die absorbierte Energie zu einer kontrollierten Tropfenbildung führt, soll die Volumenausdehnungskomponente durch den Wärmeimpuls in kürzester Zeit in die Gasphase überführt werden.

Die erfindungsgemäßen Werkstoffe zeichnen sich dadurch aus, dass sie als Volumenausdehnungskomponente mindestens ein Mono(meth)acrylat enthalten.

Als Volumenausdehnungskomponente sind niedrigsiedende flüssige Mono(meth)acrylate bevorzugt. Unter niedrig siedenden Monomeren werden Monomere mit einem Siedepunkt von weniger als 200°C bei Normaldruck verstanden. Besonders bevorzugt sind Methyl(meth)acrylat, Ethyl(meth)acrylat, Propyl(meth)acrylat, Butyl(meth)acrylat, Pentyl(meth)acrylat und Hexyl(meth)acrylat.

Volumenausdehnungskomponenten, die einen hohen Dampfdruck haben, wie z.B. von Methyl- oder Ethylmethacrylat, verdampfen nach dem Abscheiden auf der Empfängerseite und Bilden eine sauerstoffarme Gasschicht oberhalb des verdruckten Baumaterials. Das Verdunsten der Volumenausdehnungskomponente auf der Empfängerseite ist vorteilhaft, weil hierdurch die inhibierende Wirkung von Sauerstoff bei der Härtung des Baumaterials verringert oder sogar ganz vermieden wird ohne dass die die Verwendung eines Schutzgas z.B. wie Argon, Helium, Stickstoff oder CO₂ erforderlich wäre.

Andererseits können zu hohe Dampfdrücke zu Problemen auf der Trägerseite führen, wie Riss- oder Porenbildung. Diese Probleme werden durch ein zu schnelles Verdampfen der Volumenausdehnungskomponente bedingt. Erfindungsgemäß sind solche Stoffe als Volumenausdehnungskomponente bevorzugt, die bei 20°C einen Dampfdruck von maximal 60 mbar, besonders bevorzugt maximal 40 mbar aufweisen.

Die genannten Volumenausdehnungskomponenten zeichnen sich dadurch aus, dass sie bei der Aushärtung der Werkstoffe anders als nicht reaktive Volumenausdehnungskomponenten an der Reaktion teilnehmen und in das Polymernetzwerk eingebunden werden. Sie werden daher im klinischen Einsatz nicht aus dem Material herausgelöst, was im Hinblick auf die Herstellung von Dentalrestaurationen ein wesentlicher Vorteil ist.

Zusätzlich können feste, homogen dispergierte organische Substanzen als Volumenausdehnungskomponente eingesetzt werden, die sich bei Temperaturen von 80°-280°C schlagartig in Gase zersetzen, beispielsweise Azobis(isobutyronitril)(AIBN).

Der erfindungsgemäße Werkstoff enthält weiterhin einen **Photoinitiator für die radikalische Polymerisation (c),** beispielsweise einen Photopolymerisationsinitiator für den UV-Bereich, einen Photopolymerisationsinitiator für den sichtbaren Bereich oder eine Mischung davon.

Das langwelligste Absorptionsmaximum des Photopolymerisationsinitiators für den UV-Bereich liegt vorzugsweise bei einer Wellenlänge von weniger als 400 nm, insbesondere im Bereich von 300 bis weniger als 400 nm, bevorzugt im Bereich von 330 bis weniger als 400 nm, besonders bevorzugt im Bereich von 345 bis weniger als 400 nm und am meisten bevorzugt im Bereich von 360 bis weniger als 400 nm.

Das langwelligste Absorptionsmaximum des Photopolymerisationsinitiators für den sichtbaren Bereich liegt vorzugsweise bei einer Wellenlänge von mindestens 400 nm, insbesondere im Bereich von 400 bis 600 nm, besonders bevorzugt im Bereich von 400 bis 500 nm und am meisten bevorzugt im Bereich von 420 bis 480 nm.

Die Absorptionsspektren der Photopolymerisationsinitiatoren können sich in gewissen Grenzen überschneiden. Vorzugsweise beträgt die Differenz der langwelligsten Absorptionsmaxima des ersten und zweiten Photopolymerisationsinitiators mindestens 5 nm, insbesondere mindestens 10 nm, am meisten bevorzugt mindestens 15 nm. Außerdem ist es bevorzugt, dass der erste Photopolymerisationsinitiator im Wellenlängenbereich von 420 bis 750 nm und insbesondere im Wellenlängenbereich von 440 bis 700 nm einen molaren dekadischen Extinktionskoeffizienten von weniger als 10 l/(mol·cm) aufweist.

Als Photopolymerisationsinitiatoren für den UV-Bereich eignen sich insbesondere Phosphinoxide, Benzoine, Benzilketale, Acetophenone, Benzophenone, Thioxanthone sowie Mischungen davon. Besonders geeignet sind Acyl- und Bisacylphosphinoxide wie 2,4,6-Trimethylbenzoyldiphenylphosphinoxid oder Bis-(2,4,6-trimethylbenzoyl)phenylphosphinoxid, Benzoin, Benzoinalkylether, Benzildialkylketale wie Benzyldimethylketal, α-Hydroxyacetophenone wie 1-Hydroxy-cyclohexyl-phenyl-keton, 2-Hydroxy-2-methyl-1-phenyl-1-propanon oder 2-Hydroxy-1-[4-(2-hydroxyethoxy)-phenyl]-2-methyl-1-propanon, α-Dialkoxyacetophenone, α-Aminoacetophenone wie 2-Benzyl-2-(dimethylamino)-1-[4-(4-morpholinyl)-phenyl]-1-butanon oder 2-Methyl-1-[4-(methylthio)-phenyl]-2-(4-morpholinyl)-1-propanon, Alkylthioxanthone wie i-Propylthioxanthon sowie Mischungen davon. Acyl- und Bisacylphosphinoxide und insbesondere 2,4,6-Trimethylbenzoyldiphenylphosphinoxid, Bis-(2,4,6-trimethylbenzoyl)-phenylphosphinoxid und deren Mischungen sind besonders bevorzugt.

Als Photopolymerisationsinitiatoren für den sichtbaren Bereich eignen sich insbesondere α-Diketone, Acylgermanium-Verbindungen, Metallocene sowie Mischungen davon. Besonders geeignet sind α-Diketone wie Campherchinon, 9,10-Phenanthrenchinon, 1-Phenyl-propan-1,2-dion, Diacetyl, 4,4'-Dichlorbenzil oder deren Derivate, Monoacyl- und Diacylgermanium-Verbindungen wie Benzoyltrimethylgermanium, Dibenzoyldiethylgermanium oder Bis-(4-methoxybenzoyl)-diethylgermanium, Titanocene wie Bis-(η⁵-2,4-cyclopentadien-1-yl)-bis-[2,6-difluor-3-(1*H*-pyrrol-1-yl)phenyl]-titan sowie Mischungen davon. α-Diketone und insbesondere Campherchinon, 1-Phenylpropan-1,2-dion und deren Mischungen sind besonders bevorzugt. Monoacyltrialkyl- und Diacyldialkylgermanium-Verbindungen und insbesondere Benzoyltrimethylgermanium, Dibenzoyldiethylgermanium, Bis-(4-methoxybenzoyl)-diethylgermanium und deren Mischungen sind ebenfalls besonders bevorzugt. Ganz besonders bevorzugt sind auch Mischungen von mindestens einem α-Diketon und mindestens einer Acylgermanium-Verbindung.

α-Diketone werden vorzugsweise in Kombination mit Aminbeschleunigern eingesetzt. Als Aminbeschleuniger werden üblicherweise tertiäre Amine verwendet. Geeignet sind insbesondere tertiäre aromatische Amine wie N,N-Dialkyl-aniline, N,N-Dialkyl-p-toluidine oder N,N-Dialkyl-3,5-xylidine, p-(N,N-dialkylamino)-phenylethanole, p-(N,N-dialkylamino)-benzoesäurederivate, p-(N,N-dialkylamino)-benzaldehyde, p-(N,N-dialkylamino)-phenylessigsäureester oder p-(N,N-dialkylamino)-phenylpropionsäureester. Konkrete Beispiele hierfür sind N,N-Dimethylanilin, N,N-Dimethyl-p-toluidin, 3,5,N,N-Tetramethylanilin, p-(N,N-Dimethylamino)-benzaldehyd, p-(Dimethylamino)-benzoesäureethylester und p-(Dimethylamino)-benzonitril sowie Mischungen davon. Geeignet sind auch tertiäre aliphatische Amine wie Tri-n-butylamin, 2-Dimethylaminoethanol, Triethanolamin, Dimethylaminoethylmethacrylat, N,N-Dimethylbenzylamin, heterocyclische Amine wie 1,2,2,6,6-Pentamethylpiperidin, Aminosäure-Derivate wie N-Phenylglycin sowie Mischungen davon. p-(Dimethylamino)-benzoesäureethylester, Dimethylaminoethylmethacrylat, N,N-Dimethylanilin, N,N-Dimethyl-p-toluidin, Triethanolamin und deren Mischungen sind besonders bevorzugt. Dabei sind insbesondere solche Photopolymerisationsinitiatoren bevorzugt, welche bei der Einbringung von Strahlung, deren Emissionsmaximum bei einer Wellenlänge von mindestens 400 nm liegt, ausbleichen und so nach der weiteren Aushärtung keine störende Eigenfärbung mehr besitzen. Dies trifft insbesondere auf die genannten Acylgermanium-Verbindungen zu.

In einer bevorzugten Ausführungsform wird als Photopolymerisationsinitiator für den sichtbaren Bereich eine Mischung von mindestens einer Germanium-Verbindung mit mindestens einem α-Diketon in Kombination mit mindestens einem Aminbeschleuniger verwendet. Ganz besonders bevorzugte Kombinationen dieser Photopolymerisationsinitiatoren werden in EP 2 649 981 A1 beschrieben.

Erfindungsgemäß ist die Verwendung von zwei oder mehr Photoinitiatoren, die in unterschiedlichen Wellenlängenbereichen aktiv sind, bevorzugt. Der erste Photoinitiator ist dabei in dem Wellenlängenbereich aktiv, der zur Härtung der Materialien in Schritt (3) verwendet wird, der zweite Initiator in dem Wellenlängenbereich, der zur Nachvergütung in Schritt (6) verwendet wird. Bevorzugte Initiatorkombinationen werden z.B. in EP 2 751 618 A2 beschrieben.

Die **Energietransformationskomponente (d)** wird auf die Wellenlänge des zu absorbierenden Laserlichts abgestimmt. Als Energietransformationskomponente werden erfindungsgemäß anorganische und insbesondere organische Farbstoffe und Pigmente eingesetzt.

Bevorzugt sind insbesondere Farbstoffe und Pigmente, die im Wellenlängenbereich der eingesetzten Strahlenquelle, vorzugsweise Laser, absorbieren. Hier bieten sich beispielsweise für einen Neodym-YAG Laser mit einer Wellenlänge von 1064 nm folgende Farbstoffe/Pigmente besonders an: Carbon Black, Sudan Black B (CAS 4197-25-5), Bismarck Brown Y (CAS 10114-58-6), 1-Butyl-2-[2-[3-[(1-butyl-6-chlorobenz[cd]indol-2(1H)-ylidene)-ethylidene]-2-chloro-1-cyclohexen-1-yl]ethenyl]-6-chlorobenz-[cd]indolium tetrafluoroborate (CAS 155613-98-2) oder Safranin O (CAS 477-73-6). Ganz besonders bevorzugt sind Carbon Black, Sudan Black B (CAS 4197-25-5) und Safranin O (CAS 477-73-6).

Für einen grünen Laser, z.B. mit einer Wellenlänge von 532 nm, sind folgende Farbstoffe/Pigmente bevorzugt: Carbon Black, Sudan Red 7B (Oil Violet CAS 6368-72-5), Sudan IV (CAS 85-83-6), Sudan Red G (CAS 1229-55-6), Pigment Rot 144 (CAS 5280-78-4), Safranin O (CAS 477-73-6).

Für einen blauen Laser, z.B. mit einer Wellenlänge von 405 nm, sind folgende Farbstoffe/Pigmente bevorzugt: Carbon Black, Pigment Gelb 93 (CAS 5580-57-4), Sudan Yellow 146 (CAS 4314-14-1), Disperse Yellow 7 (CAS 6300-37-4).

Die Energietransformationskomponente absorbiert den Hauptteil der Energie des angebrachten Energieimpulses, beispielsweise des einfallenden Laserstrahls, und wandelt diese in Wärme um. Der so erzeugte Wärmeimpuls wird auf die Volumenausdehnungskomponente übertragen und führt zu deren schlagartigen Ausdehnung, beispielsweise zur schlagartigen Bildung von mikroskopischen Gasbläschen durch Verdampfen der Volumenausdehnungskomponente. Hierdurch wird der Transfer des Stütz- oder Baumaterials vom Trägersubstrat auf das Empfängersubstrat induziert. Das Stütz- oder Baumaterial wird auf dem Empfängersubstrat abgeschieden.

Die eingesetzte Energietransformationskomponente kann darüber hinaus auch zur gezielten Einfärbung des Werkstück eingesetzt werden.

Das erfindungsgemäße Baumaterial ist vorzugsweise so ausgelegt, dass der erzeugte Wärmeimpuls das Material lokal aufschmilzt, erweicht oder die Viskosität erniedrigt, um eine optimale Tropfenbildung zu gewährleisten.

Durch die Verwendung einer Energietransformationskomponente erübrigt sich die Verwendung von Trägerfolien, die mit absorbierenden Beschichtungen aus Titan oder anderen Stoffen versehen sind, so dass die damit verbundenen Nachteile vermieden werden können.

Neben der Energietransformationskomponente können die erfindungsgemäßen Werkstoffe vorteilhaft weitere **farbgebende Komponenten** enthalten. Als farbgebende Komponente sind anorganische und organische Pigmente bevorzugt, besonders schwermetallfreie, d.h. insbesondere Cd- und Pb-freie Pigmente. Die gebräuchlichsten anorganischen Pigmente sind solche auf der Basis der verschiedenen Eisenoxide, Chromate und Molybdate. Als organische Pigmente werden hauptsächlich Azopigmente, wie Monoazo-, Disazo-, Benzimidazolon- und Isoindolononpigmente, sowie polycyclische Pigmente, wie Phthalocyanin-, Thioindigo-, Flavanthron-, Dioxazin- und Anthantronpigmente, verwendet.

Diese Substanzklassen werden durch die Verwendung von verschiedenen Substituenten hinsichtlich der Farbnuance und der Farbtiefe modifiziert. Herstellung, Anwendung und Eigenschaften der gebräuchlichsten organischen Pigmente werden ausführlich in Herbst/Hunger, "Industrielle Organische Pigmente", VCH-Verlagsgesellschaft, Weinheim, 1987 beschrieben.

Als Pigmente eignen sich besonders Ultramarinblau, Pigmente auf der Basis von Eisenoxid, Titandioxid, Kobalt-, Aluminium-, Chrom-, Nickel-, Zirkon- und/oder Zinkoxid, Ruß und organische farbige Pigmente. Weiterhin sind organische Pigmente, wie beispielsweise rote Diazokondensationspigmente, z.B. Microlith^{®} rot BR-T (Fa. CIBA, Specialities) und gelbe Benzimidazolonpigmente, z.B. PV-Echtgelb H2G 01 (Fa. Hoechst), geeignet. Die Eisenoxidpigmente können eine rote, gelbe, braune oder schwarze Farbe aufweisen. Bevorzugte Pigmente sind schwarzes Eisenoxid, braunes Eisenoxid, gelbes organisches Pigment, rotes organisches Pigment und Titandioxid.

Die Frage, ob eine farbgebende Komponente allein zur Farbgebung oder auch als Energietransformationskomponente dient, hängt primär von der Wellenlänge des verwendeten Laserlichts ab. Stoffe, die im Wellenlängenbereich des Lasers absorbieren, sind zumindest teilweise auch als Energieabsorptionskomponente wirksam.

Die erfindungsgemäßen Baumaterialien können neben den genannten Stoffen vorzugsweise **weitere Bestandteile** enthalten, insbesondere einen oder mehrere Füllstoffe, Phasenänderungsmittel, Netzmittel, Stabilisatoren und andere Additive.

Bevorzugte **Füllstoffe** sind organische oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgröße von 0,005 bis 2 um, vorzugsweise 0,1 bis 1 um, nanopartikuläre oder mikrofeine Füllstoffe wie pyrogene Kieselsäure oder Fällungskieselsäure mit einer mittleren durchschnittlichen Partikelgröße von 5 bis 200 nm, vorzugsweise 10 bis 100 nm, Minifüllstoffe wie Quarz-,Glaskeramik- oder Glaspulver mit einer durchschnittlichen Teilchengröße von 0,01 bis 10 um, vorzugsweise 0,1 bis 1 um, sowie röntgenopake Füllstoffe wie Ytterbiumtrifluorid oder nanopartikuläres Tantal(V)-oxid bzw. Bariumsulfat mit einer mittleren durchschnittlichen Partikelgröße von 10 bis 1000 nm, vorzugsweise 100 bis 300 nm.

Wenn nicht anders angegeben handelt es sich bei allen Partikelgrößen hierin um den Mittelwert (d₅₀-Wert) der Volumenverteilung, die durch Dynamic Light Scattering für Partikel kleiner als 5 Mikrometer und durch Statische Lichtstreuung für Partikel größer als 5 Mikrometer gemessen wird. Zur Messung der Partikelgröße werden die Partikel in einer Konzentration von 0,1 Gew.-% in einer geeigneten Flüssigkeit suspendiert. Wenn die Partikel hydrolysebeständig sind (z.B. ZrO₂, Al₂O₃, ZTA, ATZ), wird deionisiertes Wasser verwendet. Der pH wird mit einer Säure oder Base so eingestellt, dass er mindestens 2, besser 3 pH-Einheiten vom isoelektrischen Punkt (Literaturwerte) der Partikel entfernt ist. Die Proben werden vor und während der Messung mit Ultraschall behandelt. Bei hydrolyseempfindlichen Partikeln (z.B. Lithiumdisikilat) wird ein Lösungsmittel verwendet, das die Partikel nicht angreift, beispielsweise Tripropylenglycol. In diesem Fall wird der pH nicht angepasst. Zur Verbesserung der Dispergierbarkeit kann ein geeignetes Oberflächenmodifizierungsmittel zugesetzt werden, beispielsweis Lubrizol^{™} Solplus D540.

Weiter bevorzugt sind sogenannte Isofüller. Hierbei handelt es sich um zerkleinerte Polymere, die vorzugsweise einen anorganischen Füllstoff enthalten. Bevorzugt sind Polymere, die durch Polymerisation der oben definierten radikalisch polymerisierbaren Bindemittel (Bindemittel a) erhalten werden. Als anorganische Füllstoffe sind die oben genannten Füllstoffe und insbesondere silanisierte, hoch disperse Kieselsäuren, Gläser und röntgenopake Füllstoffe wie Ytterbiumfluorid bevorzugt. Isofüller dienen zur Erhöhung des Füllgrads, zur Reduktion des Polymerisationsschrumpfes sowie zur Steuerung der Konsistenz und der Ästhetik der verarbeiteten Werkstoffe.

Erfindungsgemäß bevorzugte **Phasenänderungsmittel** sind Monomere mit wachsähnlichen Eigenschaften zur Einstellung der Lagerviskosität. Die Phasenänderungsmittel bewirken eine Phasenumwandlung von fest (nach dem Auftragen und Abkühlen auf dem Träger) nach flüssig (Verflüssigung durch den Wärmeeintrag des Lasers). Bevorzugte Phasenänderungsmittel sind Stearylmethacrylat, Tris(2-hydroxyethylisocyanurattrimethacrylat sowie die in DE 196 26 356 A1 beschriebenen wachsartigen polymerisierbaren Substanzen.

Unter der Lagerviskosität wird die Viskosität verstanden, die die erfindungsgemäßen Werkstoffe unter gewöhnlichen Lagerbedingungen, d.h. insbesondere bei Raumtemperatur (25°C), aufweisen. Eine hohe Viskosität bei Lagertemperatur reduziert das Sedimentieren von Pigmenten oder Füllstoffen.

Um eine homogene Mischung zu erhalten, wird das Phasenänderungsmittel vorzugsweise oberhalb seines Schmelzpunktes mit den übrigen Komponenten vermischt. Hierzu wird das Phasenänderungsmittel vorzugsweise zunächst bei Raumtemperatur mit der Hauptmasse vermengt, und die Mischung dann unter stetigem Rühren erwärmt. Alle folgenden Schritte werden bei Temperaturen oberhalb des Schmelzpunktes des Phasenänderungsmittels vorgenommen.

Erfindungsgemäß bevorzugte **Netzmittel** sind Tenside. Diese dienen zur Einstellung der Oberflächenspannung und zur Einstellung der Grenzflächenspannung zwischen Baumaterial und Träger, Stützmaterial und Empfänger und Stützmaterial und Baumaterial. Durch die Einstellung von Oberflächen- und Grenzflächenspannung wird sichergestellt, dass sich die auf den Träger aufgebrachte Schicht des Baumaterials nicht zusammenzieht (Bulging-Effekt), eine homogene Schicht auf dem Empfänger bildet und dass sich das Baumaterial nicht auf dem Stützmaterial zusammenzieht (Bulging-Effekt). Bevorzugte Tenside sind klassische ionische (z.B. Stearinsäure), amphotere (N,N,N-Trimethyl-ammonioacetat) und bevorzugt nichtionische Tenside (Polyalkylenglycolether (Fettalkoholethoxylate (FAEO)). Gewisse Tenside, besonders die oben definierten nichtionischen Tenside haben zusätzlich zur grenzflächenanpassenden Funktion auch eine Stützfunktion.

Bevorzugte **Stabilisatoren** sind Methyl-hydrochinon (MEHQ) und 2,6-Di-tert-butyl-p-kresol (BHT), Hydrochinon (HQ) und (2,2,6,6-Tetramethylpiperidin-1-yl)oxidanyl (TEMPO). Stabilisatoren dienen primär zur Verbesserung der Lagerstabilität der Werkstoffe.

Darüber hinaus können die erfindungsgemäss eingesetzten Werkstoffe weitere Additive enthalten, insbesondere Rheologiemodifizierungsmittel, wie Polyvinylalkohol, Hydroxyethylcellulose, Carboxymethylcellolose, modifizierte Fettderivate, Polyvinylpyrrolidon; Duft- und Aromastoffe, wie 2-Benzylidenheptanal (Amylzimtaldehyd), Ethyl-2-naphthylether und ätherische Öle. Als Additive kommen weiterhin Konservierungsmittel mit antimikrobieller und fungizider Wirkung wie Polyformaldehyd, Parabene wie 4-Hydroxybenzoesäuremethylester, 4-Hydroxybenzoesäurebutylester oder deren Salze, mikro- oder nanopartikuläres Silber sowie Propionsäure und ihre Salze in Betracht. Ausserdem können die erfindungsgemäßen Zusammensetzungen Lösungsmittel wie Wasser oder Ethanol oder entsprechende Lösungsmittelgemische, fluoridionenabgebende Additive, optische Aufheller und/oder Weichmacher als weitere Additive enthalten.

Zur Erzielung optimaler Druckergebnisse ist eine Abstimmung von Art und Mengen der verwendeten Komponenten in dem Werkstoff erforderlich. Die genannten Bestandteile werden vorzugsweise in den folgenden Mengen eingesetzt. Alle Angaben sind in Gew.-% und beziehen sich auf die Gesamtmasse des Werkstoffs.

| **Komponente** | **bevorzugt** | **besonders bevorzugt** | **ganz besonders bevorzugt** |
|---|---|---|---|
| Bindemittel (a) | 20-98% | 40-95% | 58-90% |
| Farbgebende Komponente | 0-10% | 0,01-5% | 0,1%-2% |
| Energietransformationskomponente (d) | 0,001-5% | 0,001-2% | 0,05-1% |
| Volumenausdehnungskomponente (b) | 0,5-15% | 1-10% | 1,5-7% |
| Phasenänderungsmittel | 0-10% | 0-8% | 0-5% |
| Füllstoff | 0-78% | 0-50% | 0-40% |
| Initiator (c) | 0,05-5% | 0,1-3% | 0,2-2% |
| Stabilisator | 0,001%-0,1% | 0,005-0,07% | 0,01-0,05% |
| Netzmittel | 0-2% | 0-1% | 0-0,5% |

Erfindungsgemäß wurde gefunden, dass sich die genannten Stoffe gut miteinander mischen lassen und homogene Zusammensetzungen ergeben. Die Mengenanteile der Komponenten lassen sich dabei in den genannten Bereichen variieren, so dass sich die Viskosität und die Oberflächenspannung gezielt einstellen lassen.

Die erfindungsgemäßen Baumaterialen haben vorzugsweise eine Viskosität von 0,025 Pas bis 1000 Pas und eine Oberflächenspannung von 20 bis 150 mN/m.

Wenn nicht anders angegeben wird die Viskosität mit einem Anton Paar Rheometer mit CP50-1 Konus-Platte-Messmittel bei einer Scherrate von 100/s und bei der Verarbeitungstemperatur gemessen. Diese beträgt bei Baumaterialien ohne Phasenänderungsmittel bevorzugt 25°C und liegt bei Baumaterialien mit Phasenänderungsmittel vorzugsweise im Bereich von 40°-70°C.

Die Oberflächen- und Grenzflächenspannung wird, wenn nicht anders angegeben, gemäß DIN 55660-1 bis -7 oder DIN 53914 (Prüfung von Tensiden Bestimmung der Oberflächenspannung) bei 25°C bestimmt.

Wesentlich für einen reproduzierbaren Druckvorgang ist die Bildung von Tröpfchen mit definierter Größe ohne die Bildung sogenannter "Satelliten". Unter Satelliten werden kleinere Tröpfchen verstanden, die zusätzlich zum "Haupttropfen" entstehen und die Druckqualität verschlechtern. Die Tröpfchenbildung wird maßgeblich durch Temperatur, Luftstrom und die Zeit zwischen dem Aufrakeln der Trägerschicht und der Tropfengenerierung beeinflusst. Für die Genauigkeit des Druckvorgangs ist weiterhin die Formstabilität der Druckmaterialschicht auf dem Empfänger unter thermischer Belastung und die Eigenschaftsveränderung von Bedeutung.

Die erfindungsgemäßen Werkstoffe werden vorzugsweise zusammen mit einem geeigneten **Stützmaterial** verdruckt. Die Stützmaterialien sollten sich in Kombination mit den verwendeten Baummaterialien bevorzugt inert verhalten. Die Baumaterialien werden zusammen, vorzugsweise sequenziell, mit dem Stützmaterial verdruckt.

Erfindungsgemäß sind Stützmaterialien bevorzugt, die in verfestigtem Zustand keine Komponenten enthalten, die mit dem verwendeten Baumaterial reagieren. Dies würde das Entfernen der Stützmaterialien aus dem Formkörper erschweren. Generell sind solche Stützmaterialien bevorzugt, die ausschließlich organische Komponenten enthalten. Nach der Härtung des Werkstücks wird das Stützmaterial wie oben beschrieben aus dem Formkörper entfernt.

Bevorzugt sind Stützmaterialien, die
(α) mindestens eine Energietransformationskomponente,
(β) mindestens eine Volumenausdehnungskomponente und
(γ) mindestens ein Bindemittel enthalten,
wobei als Bindemittel bei Raumtemperatur in reiner Form feste, nichtmetallische Substanzen bevorzugt sind. Das Bindemittel bewerkstelligt primär die Stützfunktion.

Die **Energietransformationskomponente (α)** im Stützmaterial wird auf die Wellenlänge des zu absorbierenden Laserlichts abgestimmt. Als Energietransformationskomponente sind erfindungsgemäß anorganische und insbesondere organische Farbstoffe und Pigmente bevorzugt. Bevorzugt sind insbesondere Farbstoffe und Pigmente, die im Wellenlängenbereich der eingesetzten Strahlenquelle, vorzugsweise Laser, absorbieren. Hier bieten sich beispielsweise für einen Neodym-YAG Laser mit einer Wellenlänge von 1064 nm folgende Farbstoffe/Pigmente besonders an: Carbon Black, Sudan Black B (CAS 4197-25-5), Bismarck Brown Y (CAS 10114-58-6), 1-Butyl-2-[2-[3-[(1-butyl-6-chlorobenz[cd]-indol-2(1H)-ylidene)ethylidene]-2-chloro-1-cyclohexen-1-yl]-ethenyl]-6-chlorobenz[cd]indolium tetrafluoroborate (CAS 155613-98-2) oder Safranin O (CAS 477-73-6).

Für einen grünen Laser, z.B. mit einer Wellenlänge von 532 nm, sind folgende Farbstoffe/Pigmente bevorzugt: Carbon Black, Sudan Red 7B (Oil Violet CAS 6368-72-5), Sudan IV (CAS 85-83-6), Sudan Red G (CAS 1229-55-6), Pigment Rot 144 (CAS 5280-78-4), Safranin O (CAS 477-73-6).

Für einen blauen Laser, z.B. mit einer Wellenlänge von 405 nm, sind folgende Farbstoffe/Pigmente bevorzugt: Carbon Black, Pigment Gelb 93 (CAS 5580-57-4), Sudan Yellow 146 (CAS 4314-14-1), Disperse Yellow 7 (CAS 6300-37-4).

Die **Volumenausdehnungskomponente (ß)** hat den Hauptzweck, einen Transfer des Druckmaterials vom Träger auf das Empfängersubstrat zu bewirken. Damit die absorbierte Energie zu einer kontrollierten Tropfenbildung führt, soll die Volumenausdehnungskomponente durch den Wärmeimpuls in kürzester Zeit in die Gasphase überführt werden. Als Volumenausdehnungskomponente wird vorzugsweise eine Substanz mit einem Siedepunkt von 80 - 280°C und besonders bevorzugt von 95 - 200°C eingesetzt (Siedepunkte bei Normaldruck). Bevorzugte Substanzen sind 1,8-Octandiol und 1,6-Hexandiol. Besonders bevorzugt sind bei 25°C flüssige Substanzen, insbesondere Wasser und 1-Octanol. Wasser hat den Vorteil, dass beim Verdampfen keine gesundheitsschädlichen oder explosionsgefährlichen Lösungsmitteldämpfe entstehen.

Weitere bevorzugte Stoffe, die als Volumenausdehnungskomponente (β) eingesetzt werden können, sind Propylenglycoldiacetat, Ethylenglycoldiacetat, Triethyl-2-acetylcitrate, Triethylcictrat, Adibinsäuredimethylester, Adipinsäurediethylester, Triethylenglycol, Glutarsäurediethylester, Glutarsäuredimethylester, Diethylsuccinate, Essigsäurebutylester, Essigsäure-n-hexylester. Die Volumenausdehnungskomponente wird vorzugsweise so auf das verwendete Bindemittel abgestimmt, dass die Viskosität, die Oberflächenspannung und die Homogenität in den hierin definierten Bereichen liegen. Eine erfindungsgemäß geeignete Homogenität ist dann gegeben, wenn keine sichtbare Phasentrennung vorliegt. Hierzu werden polare Bindemittel wie PEG, PVA vorzugsweise mit einer polaren Volumenausdehnungskomponente wie z.B. Wasser und apolare Bindemittel wie Paraffin mit einer weniger polaren Volumenausdehnungskomponenten wie 1-Octanol kombiniert.

Alternativ können feste, homogen dispergierte organische Substanzen als Volumenausdehnungskomponente (β) eingesetzt werden, die sich bei Temperaturen von 80°-280°C schlagartig in Gase zersetzen, beispielsweise Azobis(isobutyronitril) (AIBN).

Als **Bindemittel (γ)** werden vorzugsweise Polymere, Wachse und/oder nichtionische Tenside eingesetzt, die bei einer Temperatur von unter 40°C fest sind.

Erfindungsgemäß bevorzugte Polymere sind Glykolpolymere, insbesondere Polyethylenglykol (PEG), Polypropylenglykol (PPG), PEG-PPG-Copolymere und PVA. Ganz besonders bevorzugt ist Polyethylenglykol (PEG) mit einem Molekulargewicht von 1500-10000g/mol. Weiter bevorzugt sind vernetzte Polymere wie Polyacrylamid, Polyvinylpyrrolidon, Amylopektin, Gelatine, Cellulose, Polymere auf der Basis von Polyacrylsäure und insbesondere Copolymere von Acrylsäure oder Natriumacrylat mit Acrylamid. Diese vernetzten Polymere sind polar und können Hydrogele bilden. Polare Polymere eignen sich besonders zur Kombination mit einer polaren Volumenausdehnungskomponente wie Wasser.

Der Begriff "Wachs" ist so zu verstehen, wie er von der Deutschen Gesellschaft für Fettwissenschaft in der DGF-Einheitsmethode MI1 (75) festgelegt wurde. Da die chemische Zusammensetzung und Herkunft verschiedener Wachse sehr unterschiedlich ist, werden Wachse über ihre mechanischphysikalischen Eigenschaften definiert. Ein Stoff wird als Wachs bezeichnet, wenn er bei 20°C knetbar, fest bis brüchig hart, eine grobe bis feinkristalline Struktur aufweist, farblich durchscheinend bis opak, jedoch nicht glasartig ist; über 40°C ohne Zersetzung schmilzt, schon wenig oberhalb des Schmelzpunktes leicht flüssig (niedrigviskos) und nicht fadenziehend ist; eine stark temperaturabhängige Konsistenz und Löslichkeit aufweist, sowie unter leichtem Druck polierbar ist. Typischerweise gehen Wachse zwischen 40°C und 130°C in den schmelzflüssigen Zustand über; Wachse sind in der Regel wasserunlöslich. Vorzugsweise haben Wachse zur Verwendung in dem erfindungsgemäßen Stützmaterial einen Schmelzpunkt im Bereich von 40 bis kleiner 80°C, besonders bevorzugt von 45 bis 65°C.

Je nach ihrer Herkunft teilt man Wachse in drei Hauptgruppen ein, nämlich natürliche Wachse, wobei hier wiederum zwischen pflanzlichen und tierischen Wachsen, Mineralwachsen und petrochemischen Wachsen unterschieden wird; chemisch modifizierte Wachse und synthetische Wachse. Das als Bindemittel in dem erfindungsgemäßen Stützmaterial eingesetzte Wachs kann aus einer Wachsart oder auch aus Mischungen verschiedener Wachsarten bestehen.

Bevorzugt sind petrochemische Wachse, wie etwa Paraffinwachs (Hartparaffin), Petrolatum, Mikrowachs (Mikroparaffin) und deren Mischungen, besonders bevorzugt ist Paraffinwachs. Gut geeignet sind Paraffinwachse, die als Spritzgussbinder zur Fertigung von oxidkeramischen und nichtoxidkeramischen Bauteilen im Heißgießverfahren (Niederdruckspritzguss) kommerziell erhältlich sind, z.B. Paraffinwachs mit einem Schmelzpunkt ca. 54 - 56°C, einer Viskosität von 3 - 4 mPa-s bei 80°C. Häufig enthalten kommerziell erhältliche Wachse bereits Emulgatoren und/oder weitere Komponenten zur Anpassung der Rheologie.

Als Wachs können auch pflanzliche Wachse, z.B. Candelillawachs, Carnaubawachs, Japanwachs, Espartograswachs, Korkwachs, Guarumawachs, Reiskeimölwachs, Zuckerrohrwachs, Ouricurywachs, Montanwachs; tierische Wachse, z.B. Bienenwachs, Schellakwachs, Walrat, Lanolin (Wollwachs), Bürzelfett; Mineralwachse, z.B. Ceresin, Ozokerit (Erdwachs); chemisch modifizierte Wachse, z.B. Montanesterwachse, Sasolwachse, hydrierte Jojobawachse, oder synthetische Wachse, z.B. Polyalkylenwachse, Polyethylenglykolwachse, verwendet werden.

Nichtionische Tenside sind Stoffe mit grenzflächenaktiven Eigenschaften, die in wässrigen Medien keine Ionen bilden. Es handelt sich um Moleküle, die einen hydrophoben und einen hydrophilen Teil aufweisen. Durch Wahl der Länge und Art der hydrophoben und hydrophilen Teile kann die Gesamt-Hydrophobizität der Moleküle eingestellt werden.

Bevorzugt sind Stützmaterialien, die als nichtionisches Tensid (γ) ein Tensid mit einem Schmelzpunkt von 40°C bis 120°C, bevorzugt 45°C bis 80°C enthalten.

Bevorzugte nichtionische Tenside sind die Ethoxylate von Fettalkoholen, Oxoalkoholen oder Fettsäuren, Fettsäureester von Zuckern und hydrierten Zuckern, Alkylglycoside sowie Blockpolymere des Ethylen- und Propylenoxids, insbesondere kurzkettige Block-Co-Oligomere.

Besonders bevorzugt sind Fettsäuresester von hydrierten Zuckern, insbesondere solche mit der Formel R'-CO-O-Zucker, wobei R' ein verzweigter oder bevorzugt gerader Alkylrest mit 10 bis 25 Kohlenstoffatomen, vorzugsweise 12 bis 22 Kohlenstoffatomen ist. Bevorzugt sind gerade Alkylreste mit 15 bis 22 Kohlenstoffatomen. "Zucker" steht für einen hydrierten Zuckerrest, der vorzugsweise 0- bis 5-fach ethoxyliert ist. Ganz besonders bevorzugt sind Fettsäureester des Sorbitols, insbesondere Sorbitanstearate wie z.B. Sorbitanmonostearat (CAS 1338-41-6) .

Eine weitere bevorzugte Gruppe von Tensiden sind Ethoxylate von Fettsäuren, insbesondere solche mit der allgemeinen Formel R"- (CO) - (OCH₂CH₂) m-OH, in der R" ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. m ist eine ganze Zahl von 0 bis 20, bevorzugt 0 bis 10 und besonders bevorzugt 0 bis 6.

Ganz besonders bevorzugte Tenside (γ) sind Fettalkohole und Ethoxylate von Fettalkoholen, insbesondere Polyalkylenglycolether mit der allgemeinen Formel R-(OCH₂CH₂)n-OH, in der R ein Alkylrest mit 10 bis 20 Kohlenstoffatomen und n eine ganze Zahl von 0 bis 25 ist. R kann ein verzweigter oder bevorzugt gerader Alkylrest sein, wobei Alkylreste mit 12 bis 25 Kohlenstoffatomen und besonders gerade Alkylreste mit 12 bis 22 Kohlenstoffatomen bevorzugt sind. Ganz besonders bevorzugte Alkylreste sind Lauryl, Cetyl, Cetearyl und Stearyl. Die Polyalkylenglycolether sind durch Umsetzung der entsprechenden Fettalkohole mit Ethylenoxid (EO) erhältlich. Der Index n gibt die Anzahl an Ethylenoxidresten an. Polyalkylenglycolether mit 0 bis 21 (n = 2-21), insbesondere 0 bis 12 (n = 2-12) und ganz besonders 0 bis 5 (n = 2-5) Ethylenoxidresten sind bevorzugt. Beispiele für erfindungsgemäß bevorzugte Polyalkylenglycolether sind Verbindungen, in denen R ein Cetylrest (C₁₆-Rest)und n 20 und insbesondere 2 ist. Diese Verbindungen haben die INCI-Bezeichnungen Ceteth-2 und Ceteth-20. Ceteth-2 hat z.B. die Formel C₁₆H₃₃- (OCH₂CH₂)₂-OH. Weiter bevorzugt sind Verbindungen, in denen R ein Stearylrest (C₁₈-Rest) und n 2, 10, 20 oder 21 ist. Diese Verbindungen haben die INCI-Bezeichnungen Steareth-2, Steareth-10, Steareth-20 und Steareth-21. Steareth-2 hat z.B. die Formel C₁₈H₃₇-(OCH₂CH₂)₂-OH.

Ganz besonders bevorzugte nichtionische Tenside sind Steareth-20, Steareth-10, Ceteth-20 und insbesondere Steareth-2 und Ceteth-2. Mischungen unterschiedlicher nichtionischer Tenside und insbesondere unterschiedlicher Polyalkylenglycolether können ebenfalls verwendet werden.

Bevorzugt sind Bindemittel (γ) mit einem Schmelzpunkt zwischen 40°C bis 200°C, besonders bevorzugt 50°C bis 80°C, wobei solche Bindemittel besonders bevorzugt sind, die sich beim Schmelzen nicht thermisch zersetzen. Im geschmolzenen Zustand hat das Bindemittels vorzugsweise eine Viskosität von unter 100 Pas, besonders bevorzugt unter 20 Pas und ganz besonders bevorzugt unter 5 Pas, damit es gut vom Bauteil entfernbar ist. Das Bindemittel sollte möglichst rückstandsfrei verbrennbar sein. Wichtig ist eine ausreichende Festigkeit des Stützmaterials im festen Zustand, um das Druckmaterial entsprechend unterstützen zu können.

Die erfindungsgemäß bevorzugten Stützmaterialien können zur Einstellung der Oberflächenspannung und zur Einstellung der Grenzflächenspannung zwischen Stützmaterial und Träger, Stützmaterial und Empfänger und Stützmaterial und Baumaterial zusätzlich ein oder mehrere weitere Tenside enthalten. Durch die Einstellung von Oberflächen- und Grenzflächenspannung wird sichergestellt, dass sich die auf den Träger aufgebrachte Schicht des Stützmaterials nicht zusammenzieht (Bulging-Effekt), eine homogene Schicht auf dem Empfänger bildet und dass sich das Baumaterial nicht auf dem Stützmaterial zusammenzieht (Bulging-Effekt). Bevorzugte Tenside zur Einstellung der Ober- und Grenzflächenspannung sind ionische Tenside (z.B. Stearinsäure), amphotere Tenside (z.B. N,N,N-Trimethylammonioacetat) und bevorzugt die oben genannten nichtionischen Tenside, wobei hier Fettalkoholethoxylate (FAEO) und Polyalkylenglycolether besonders bevorzugt sind. Gewisse Tenside, besonders die oben definierten nichtionischen Tenside haben zusätzlich zur grenzflächenanpassenden Funktion auch eine Stützfunktion.

Die erfindungsgemäßen bevorzugten Stützmaterialen haben vorzugsweise eine Viskosität von 0,2 Pas bis 1000 Pas und eine Oberflächenspannung von 20 bis 150 mN/m, bevorzugt 30 bis 100 mN/m und besonders bevorzugt 40 bis 90 mN/m.

Die erfindungsgemäß bevorzugten Stützmaterialien können neben den genannten Stoffen vorzugsweise ein oder mehrere Additive enthalten. Bevorzugte Additive sind Stabilisatoren wie Methyl-hydrochinon (MEHQ) und 2,6-Di-tert-butyl-p-kresol (BHT); Rheologiemodifizierungsmittel wie, Polyvinylalkohol, Hydroxyethylcellulose, Carboxymethylcellolose, Polyvinylpyrrolidon; Duftstoffe, wie 2-Benzylidenheptanal (Amylzimtaldehyd), Ethyl-2-naphthylether und ätherische Öle; und Füllstoffe. Bevorzugt sind organische Füllstoffe, die rückstandsfrei verbrennen. Als Additive kommen weiterhin antimikrobielle Stoffe wie Polyformaldehyd, Parabene wie Hydroxybenzoesäuremethylester in Betracht.

Die erfindungsgemäß bevorzugten Stützmaterialien enthalten vorzugsweise:
0,05 bis 30 Gew.-%, besonders bevorzugt 0,05 bis 20 Gew-% Energietransformationskomponente (α),
5 bis 60 Gew.-%, besonders bevorzugt 8 bis 50 Gew-% Volumenausdehnungskomponente (β),
35 bis 94,95 Gew.-%, besonders bevorzugt 40 bis 90 Gew-% und ganz besonders bevorzugt 49 bis 90 Gew.-% Bindemittel (γ)

Alle Mengenangaben beziehen sich, wenn nicht anders angegeben, auf die Gesamtmasse des Stützmaterials.

Das erfindungsgemäße Verfahren eignet sich zur Herstellung von Formkörpern aus gefüllten und ungefüllten Kunststoffen.

Ein Vorteil des erfindungsgemäßen LIFT-Verfahrens ist, dass unterschiedlichste Materialien selektiv aufgetragen und im Anschluss an den Materialauftrag ihren Eigenschaften entsprechend eigenständig ausgehärten bzw. sich verfestigen oder durch einen zusätzlichen Prozessschritt ausgehärtet bzw. verfestigt werden können. Mit dem Verfahren können Materialien mit hoher Viskosität und hohen Füllstoffgehalten verarbeitet werden, so dass sich Bauteile mit ausgezeichneten physikalischen Eigenschaften, d.h. mit hoher Zähigkeit, hoher Bruchfestigkeit, guter Abrasionsbeständigkeit und hohem E-Modul herstellen lassen. Außerdem könnten Füllstoffe mit größeren Partikelgrößen eingesetzt werden als dies beim Inkjet-Verfahren möglich ist. Durch das Verfahren können gezielt einzelne Bereiche des Bauteils mit unterschiedlich zusammengesetzten Werkstoffen, beispielsweise unterschiedlich eingefärbte Materialien, hergestellt werden, so dass sich Dentalrestaurationen mit sehr natürlichem Aussehen herstellen lassen.

Im Folgenden wird die Erfindung anhand von Ausführungsbeispielen näher erläutert

### Ausführungsbeispiele

### Beispiele 1 bis 3: Herstellung von Baumaterialien

Durch Mischen der Komponenten wurden die in der folgenden Tabelle 1 angegebenen Werkstoffe hergestellt. Der Werkstoff aus Beispiel 1 eignet sich besonders zur Herstellung von partiellen und totalen abnehmbaren Prothesen (Prothesenbasismaterial), der Werkstoff aus Beispiel 2 zur Herstellung von Aufbissschienen für die Kieferorthopädie (orthodontisches Material) und der Werkstoff aus Beispiel 3 zur Herstellung von künstlichen Zähnen für die abnehmbare Prothetik (Zahnmaterial) .

Zur Herstellung der Werkstoffe wurden Bindemittel, Stabilisator und ggf. Netzmittel mit einem Flügelrührwerk homogen gemischt und solange gerührt, bis der Stabilisator vollständig gelöst war. Die Mischungen wurden zur Erniedrigung der Viskosität zum Teil auf maximal 70°C erwärmt. Anschließend wurde ein kleiner Teil dieser Mischung mit Farbpigmenten und der Energietransformationskomponente vermengt. Die Pigmente wurden auf einem Dreiwalzenstuhl aufgeschlossen und solange dispergiert, bis eine homogen eingefärbte Mischung erhalten wurde. Diese Mischung wurde dann zur Hauptmasse der Ausgangsmischung gegeben und solange gerührt, bis eine homogene Mischung entstanden war.

Füllstoffe wurden ggf. von Hand in die Massen gerührt und dann mit einem Dreiwalzenstuhl oder einem Planetenmischer homogenisiert.

Die Photoinitiatoren wurden in der Volumenausdehnungskomponente unter Ausschluss von kurzwelligem Licht (< 550nm) vollständig gelöst, diese Mischung dann der vorbereiteten Hauptmasse zugegeben und mit einem Rührwerk, einem Dreiwalzenstuhl oder einem Planetenmischer homogen vermischt. Die Mischungen waren mit dem LIFT-Verfahren verarbeitbar.

**Tabelle 1: Erfindungsgemäße Dentalwerkstoffe**

| **Komponente** | **Bsp. 1** | **Bsp. 2** | **Bsp. 3** |
|---|---|---|---|
| **Bindemittel (a)** | | | |
| Bis-GMA¹⁾ | 39.5 | 35.0 | 15.0 |
| UDMA²⁾ | 30.0 | 32.0 | 12.0 |
| EGDMA³⁾ | 25.0 | - | 9.0 |
| D3MA⁴⁾ | - | 22.0 | - |

| Farbgebende Komponente | | | |
|---|---|---|---|
| PV Fast Red HF4 B¹⁵⁾ | 0.3 | - | - |
| Microlith Gelb 3G-K⁵⁾ | - | - | 0.18 |

| **reaktive Volumenausdehnungskomponente (b)** | | | |
|---|---|---|---|
| Butylmethacrylat⁶⁾ | 4.0 | 10.0 | 4.0 |

| **Füllstoff** | | | |
|---|---|---|---|
| Pyrogene Kieselsäure⁷⁾ | - | - | 15.0 |
| Ytterbiumfluorid⁸⁾ | - | - | 14.0 |
| Isofüller⁹⁾ | - | - | 30.0 |

| **Energietransformationskomponente (d)** | | | |
|---|---|---|---|
| Kupferphthalocyanin¹⁰⁾ | 0.1 | 0.1 | - |

| **Komponente** | **Bsp. 1** | **Bsp. 2** | **Bsp. 3** |
|---|---|---|---|
| **Photoinitiator (c)** | | | |
| Campherchinon DL¹¹⁾ | 0.3 | 0.2 | 0.12 |
| Ethyl-4 (dimethylamino) benzoat¹²⁾ | 0.5 | 0.4 | 0.20 |

| **UV-Stabilisator** | | | |
|---|---|---|---|
| 2-(2'-Hydroxy-5'-methylphenyl)-benzotriazol¹³⁾ | 0.3 | 0.3 | 0.10 |

| **Netzmittel** | | | |
|---|---|---|---|
| Posphorsäure-Polyester¹⁴⁾ | - | - | 0.40 |

| | | | |
|---|---|---|---|
| ²⁾ 2,2-Bis[4-(2-hydroxy-3-methacryloxypropoxy)phenyl]propan (CAS 1565-94-2) ²⁾ 1,6-Bis[2-methacryloyloxyethoxycarbonylamino]-2,4,4-trimethyl-hexan (CAS 72869-86-4) ³⁾ Ethylenglycoldimethacrylat (CAS 97-90-5) ⁴⁾ 1,10-Decanediol-dimethacrylat (CAS 6701-13-9) ⁵⁾ CAS 5580-57-4 ⁶⁾ CAS 97-88-1 ⁷⁾ Aerosil OX50 (CAS 7631-86-9n) ⁸⁾ CAS 13760-80-0 ⁹⁾ Splitterpolymerisat (zu Pulver gemahlenes Mikrofüllerkomposit) ¹⁰⁾ Heliogen Blau L7072D (CAS 147-14-8) ¹¹⁾ CAS 10373-78-1 ¹²⁾ Quantacure EPD (CAS 10287-53-3) ¹³⁾ Chisorb P (CAS2440-22-4) ¹⁴⁾ Phosphorsäurepolyester (72243-070628, Germany) ¹⁵⁾ CAS 59487-23-9 | | | |

### Beispiel 4: Herstellung von Bauteilen durch LIFT-Prozess

Die Materialien aus den Beispielen 1 bis 3 und das Stützmaterial wurden separat mit einer Rakel auf eine plasmabehandelte 50 µm dicke PET-Folie aufgerakelt. Die Materialfilmdicke betrug in allen Fällen durchschnittlich 30 µm.

Die Trägersubstrate wurden in den Arbeitsbereich des Lasers transferiert und dort innerhalb von maximal 5 Sekunden nach dem Aufrakeln verarbeitet. Als Laser wurde ein Neodym-YAG Laser mit einer Wellenlänge von 1064 nm verwendet. Die beschichteten Träger wurden von hinten durch das Trägersubstrat hindurch mit einem Laserpuls von 100 ns mit einer Leistung von 12 mW beschossen, wobei der Laserstrahl auf einen Punkt mit einem Durchmesser von 50 µm fokussiert war. Als Empfängersubstrat wurden plasmabehandelte PET-Folien mit einer Dicke von 50 µm verwendet. Die Tropfen wurden mit einer Überlappung von 0-30µm nebeneinander auf dem Empfängersubstrat abgeschieden, während der Materialfilm auf dem Träger kontinuierlich erneuert wurde. Der Abstand zwischen Trägersubstrat (Tropfengenerierungsort, d.h. der Stelle an der der Laser die Tropfen aus der Materialschicht herausschießt) und Empfängersubstrat betrug 300 µm.

Auf dem Empfängersubstrat wurden die Materialien 1 bis 3 unter einem konstanten Gasfluss von trockenem Sticksoff mittels einer LED-Blaulichtquelle während 10 Sekunden bei 460 nm mit einer Leistungsdichte von 50mW/cm2 ausgehärtet.

Das Stützmaterial wurde ortsselektiv auf das Empfängersubstrat aufgetragen. In die nicht mit Stützmaterial bedrucken Freiräume wurde das Baumaterial auf die beschriebene Weise aufgebracht. Die Verfestigung des Stützmaterials erfolgte durch Trocknen, indem ein konstanter Luftfluss für 10 Sekunden über das Empfängersubstrat geleitet wurde. Dadurch wurde das Stützmaterial soweit getrocknet, dass es erstarrte. Es wurden jeweils 5 Schichten Stützmaterial und 5 Schichten Baumaterial abgeschieden, dann wurde das Bauteil mit einer Hartmetallfräse mit Materialabsaugung zur Anpassung der Schichthöhe geglättet. Nach dem Glätten wurden weitere Schichten aufgebracht und erneut geglättet. Der Vorgang wurde so lange wiederholt, bis das Objekt fertig gedruckt war.

Das verwendete Stützmaterial hatte die folgende Zusammensetzung:

| ***Komponente*** | ***Anteil*** |
|---|---|
| Deionisiertes Wasser | 49,80 Gew.-% |
| Polyethylenglycol 2000 g/mol | 50 Gew.-% |
| Safranin O (CAS 477-73-6) | 0,20 Gew.-% |

Nach dem Verdrucken der Stütz- und Baumaterialien wurden die Bauteile aus der Baukammer entnommen, das Stützmaterial mit einer weichen Zahnbürste und handwarmem Wasser mechanisch entfernt und die Werkstücke dann im Lumamat (Ivoclar Vivadent AG) mit Programm 2 für 11 Minuten nachbelichtet und dadurch vollständig ausgehärtet.

Danach wurden mit einem Dentalschleifer (Handstück) mit Polierscheibe und Polierpaste die Oberflächen der Bauteile poliert.

## Patentansprüche

1. Verwendung eines Werkstoffs, der
(a) mindestens ein mono- oder multifunktionelles (Meth)acrylat oder eine Mischung davon als polymerisierbares Bindemittel,
(b) mindestens ein Mono(meth)acrylat als Volumenausdehnungskomponente,
(c) mindestens einen Photoinitiator für die radikalische Polymerisation und
(d) mindestens einen anorganischen oder organischen Farbstoff und/oder mindestens ein anorganisches oder organisches Pigment als Energietransformationskomponente enthält,
als Baumaterial für den Energieimpuls induzierten Transferdruck (LIFT).

2. Verwendung nach Anspruch 1, wobei der Werkstoff als Volumenausdehnungskomponente (b) Methyl(meth)acrylat, Ethyl(meth)acrylat, Propyl(meth)acrylat, Butyl(meth)-acrylat, Pentyl(meth)acrylat, Hexyl(meth)acrylat und/oder N,N-Dimethylacrylamid enthält.

3. Verwendung nach einem der vorhergehenden Ansprüche, wobei der Werkstoff als Photoinitiator (c)
einen Initiator für den UV-Bereich, vorzugsweise ein Phosphinoxid, Benzoin, Benzilketal, Acetophenon, Benzophenon, Thioxanthon oder eine Mischung davon, ein Acyl- und Bisacylphosphinoxid wie 2,4,6-Trimethylbenzoyldiphenylphosphinoxid oder Bis-(2,4,6-trimethylbenzoyl)phenylphosphinoxid, Benzoin, einen Benzoinalkylether, ein Benzildialkylketal wie Benzyldimethylketal, ein α-Hydroxyacetophenon wie 1-Hydroxy-cyclohexyl-phenyl-keton, 2-Hydroxy-2-methyl-1-phenyl-1-propanon oder 2-Hydroxy-1-[4-(2-hydroxyethoxy)-phenyl]-2-methyl-1-propanon, ein α-Dialkoxyacetophenon, ein α-Aminoacetophenon wie 2-Benzyl-2-(dimethylamino)-1-[4-(4-morpholinyl)-phenyl]-1-butanon oder 2-Methyl-1-[4-(methylthio)-phenyl]-2-(4-morpholinyl)-1-propanon, ein Alkylthioxanthon wie i-Propylthioxanthon oder eine Mischung davon, ein Acyl- und Bisacylphosphinoxid, 2,4,6-Trimethylbenzoyldiphenylphosphinoxid, Bis-(2,4,6-trimethylbenzoyl)-phenylphosphinoxid oder eine Mischung davon,
und/oder
einen Photoinitiator für den sichtbaren Bereich enthält, vorzugsweise ein α-Diketon, eine Acylgermanium-Verbindung, ein Metallocen oder eine Mischung davon, ein α-Diketon wie Campherchinon, 9,10-Phenanthrenchinon, 1-Phenyl-propan-1,2-dion, Diacetyl, 4,4'-Dichlorbenzil oder ein Derivat davon, ein Monoacyl- und Diacylgermanium-Verbindung wie Benzoyltrimethylgermanium, Dibenzoyldiethylgermanium oder Bis-(4-methoxybenzoyl)-diethylgermanium, ein Titanocen wie Bis-(η^{s}-2,4-cyclopentadien-1-yl)-bis-[2,6-difluor-3-(1*H-*pyrrol-1-yl)phenyl]-titan oder eine Mischung davon, ein α-Diketon wie Campherchinon, 1-Phenylpropan-1,2-dion oder eine Mischung davon, eine Monoacyltrialkyl- oder Diacyldialkylgermanium-Verbindung, Benzoyltrimethylgermanium, Dibenzoyldiethylgermanium, Bis-(4-methoxybenzoyl)-diethylgermanium oder eine Mischungen davon, oder eine Mischungen von mindestens einem α-Diketon und mindestens einer Acylgermanium-Verbindung.

4. Verwendung nach einem der vorhergehenden Ansprüche, wobei der Werkstoff zusätzlich mindestens eine farbgebende Komponente enthält, vorzugsweise mindestens ein anorganisches Pigment auf der Basis von Eisenoxid, Chromat oder Molybdat, und/oder mindestens ein organisches Pigment, das aus Azopigmenten, wie Monoazo-, Disazo-, Benzimidazolon- und Isoindolononpigmenten, polycyclischen Pigmenten, wie Phthalocyanin-, Thioindigo-, Flavanthron-, Dioxazin- und Anthantronpigmenten ausgewählt ist.

5. Verwendung nach einem der vorhergehenden Ansprüche, der zusätzlich mindestens einen Füllstoff, ein Phasenänderungsmittel, ein Netzmittel und/oder einen Stabilisator enthält.

6. Verwendung nach einem der vorhergehenden Ansprüche, wobei der Werkstoff
20 bis 98 Gew.-%, bevorzugt 40 bis 95 Gew-%, besonders bevorzugt 58 bis 90 Gew.-% Bindemittel (a),
0,5 bis 15 Gew.-%, bevorzugt 1 bis 10 Gew-%, besonders bevorzugt 1,5 bis 7 Gew.-% Volumenausdehnungskomponente (b),
0,05 bis 5 Gew.-%, bevorzugt 0,1 bis 3 Gew-%, besonders bevorzugt 0,2 bis 2 Gew.-% Initiator (c) und
0,001 bis 5 Gew.-%, bevorzugt 0,001 bis 2 Gew-%, besonders bevorzugt 0,05 bis 1 Gew.-% Energietransformationskomponente (d)
enthält, jeweils bezogen auf die Gesamtmasse des Werkstoffs.

7. Verwendung nach Anspruch 6, wobei der Werkstoff zusätzlich
0 bis 10 Gew.-%, bevorzugt 0 bis 8 Gew-%, besonders bevorzugt 0 bis 5 Gew.-% Phasenänderungsmittel und/oder
0 bis 78 Gew.-%, bevorzugt 0 bis 50 Gew-%, besonders bevorzugt 0 bis 40 Gew.-% Füllstoff und/oder
0,001 bis 0,1 Gew.-%, bevorzugt 0,005 bis 0,07 Gew-%, besonders bevorzugt 0,01 bis 0,05 Gew.-% Stabilisator und/oder
0 bis 2 Gew.-%, bevorzugt 0 bis 1 Gew-%, besonders bevorzugt 0 bis 0,5 Gew.-% Netzmittel
enthält, jeweils bezogen auf die Gesamtmasse des Werkstoffs.

8. Verfahren zur generativen Herstellung dreidimensionaler Objekte, das die folgenden Schritte umfasst:
(1) flächiges Aufbringen eines Stütz-/Baumaterials auf einen Träger in definierter Schichtdicke, vorzugsweise in einer Schichtdicke von 3 - 300 µm, besonders bevorzugt 10 - 100 µm,
(2) Transfer eines Anteils des Stütz-/Baumaterials vom Trägersubstrat (Donor) auf ein Empfängersubstrat (Akzeptor) durch den lokalen, ortsselektiven Eintrag eines Energieimpulses, vorzugsweise eines Laserimpulses,
(3) Verfestigen des Stütz-/Baumaterials auf dem Empfängersubstrat, vorzugsweise durch Trocknen, Strahlenhärtung oder Ändern des Aggregatszustandes,
(4) Wiederholen der Schritte (1)-(3) bis das gewünschte Objekt erstellt ist,
(5) ggf. Entfernen des Stützmaterials und optionales Reinigung des Objekts,
(6) optionales Nachvergüten des Bauteils durch weiteres Härten, vorzugsweise durch Trocknung, Strahlung, Wärme oder eine Kombination davon,
(7) optionale mechanische Bearbeitung des Objekts durch z.B. Trowalisieren oder manuelles Bearbeiten wie Schleifen oder Polieren,
**dadurch gekennzeichnet, dass** man als Baumaterial einen Werkstoff wie in einem der Ansprüche 1 bis 7 definiert einsetzt.

9. Verfahren nach Anspruch 8, bei dem man das Stütz-/Baumaterial im Anschluss an Schritt (3) glättet, vorzugsweise mit einer Walze, Klinge, Fräse und/oder einem Abstreifer.

10. Verfahren nach Anspruch 8 oder 9, bei dem man in Schritt (2) eine oder mehrere Schichten Stützmaterial auf das Empfängersubstrat aufbringt und man dann auf oder in die zuvor abgeschiedenen Schichten des Stützmaterials mittels LIFT ein Baumaterial abscheidet/aufträgt.

11. Verfahren nach einem der Ansprüche 8 bis 10, bei dem man als Träger in Schritt (1) und oder Empfängersubstrat in Schritt (2) eine Polymerfolie, einen Glasträger, einen Träger aus einem nicht-metallischen, anorganischen, nicht-porösen Werkstoff, einen metallischen Träger oder einen keramischen Träger einsetzt.

12. Verfahren nach Anspruch 11, bei dem man in Schritt (2) ein nicht poröses Empfängersubstrat einsetzt.

13. Verfahren nach einem der Ansprüche 8 bis 12, bei dem der Energieeintrag in Schritt (2) über die dem Stütz- oder Baumaterial abgewandte Seite des Trägersubstrats erfolgt.

## Claims

1. Use of a material, which comprises
(a) at least one mono- or multifunctional (meth)acrylate or a mixture thereof as polymerizable binder,
(b) at least one mono(meth)acrylate as volume expansion component,
(c) at least one photoinitiator for the radical polymerization, and
(d) at least one inorganic or organic dye and/or at least one inorganic or organic pigment as energy transformation component,
for use as construction material for energy-pulse-induced transfer printing (LIFT).

2. The use according to claim 1, wherein the material comprises as the volume expansion component (b) methyl (meth)acrylate, ethyl (meth)acrylate, propyl (meth)acrylate, butyl (meth)acrylate, pentyl (meth)acrylate, hexyl (meth)acrylate and/or N,N-dimethylacrylamide.

3. The use according to one of the preceding claims, wherein the material comprises as the photoinitiator
an initiator for the UV range, preferably a phosphine oxide, benzoin, benzil ketal, acetophenone, benzophenone, thioxanthone or a mixture thereof, an acyl- and bisacylphosphine oxide such as 2,4,6-trimethylbenzoyldiphenylphosphine oxide or bis-(2,4,6-trimethylbenzoyl)phenylphosphine oxide, benzoin, a benzoin alkyl ether, a benzil dialkyl ketal such as benzyl dimethyl ketal, an α-hydroxyacetophenone such as 1-hydroxy-cyclohexyl-phenyl-ketone, 2-hydroxy-2-methyl-1-phenyl-1-propanone or 2-hydroxy-1-[4-(2-hydroxyethoxy)-phenyl]-2-methyl-1-propanone, an α-dialkoxyaceto-phenone, an α-aminoacetophenone such as 2-benzyl-2-(dimethylamino)-1-[4-(4-morpholinyl)-phenyl]-1-butanone or 2-methyl-1-[4-(methylthio)-phenyl]-2-(4-morpholinyl)-1-propanone, an alkylthioxanthone such as i-propylthioxanthone or a mixture thereof, an acyl- and bisacylphosphine oxide, 2,4,6-trimethylbenzoyldiphenylphosphine oxide, bis-(2,4,6-trimethylbenzoyl)-phenylphosphine oxide or a mixture thereof,
and/or
a photoinitiator for the visible range, preferably an α-diketone, an acylgermanium compound, a metallocene or a mixture thereof, an α-diketone such as camphorquinone, 9,10-phenanthrenequinone, 1-phenyl-propane-1,2-dione, diacetyl, 4,4'-dichlorobenzil or a derivative thereof, a monoacyl- and diacylgermanium compound such as benzoyltrimethylgermanium, dibenzoyldiethylgermanium or bis-(4-methoxybenzoyl)-diethylgermanium, a titanocene such as bis-(η^{s}-2,4-cyclopentadien-1-yl)-bis-[2,6-difluoro-3-(1/7-pyrrol-1-yl)phenyl]-titanium or a mixture thereof, an α-diketone such as camphorquinone, 1-phenylpropane-1,2-dione or a mixture thereof, a monoacyltrialkyl- or diacyldialkylgermanium compound, benzoyltrimethylgermanium, dibenzoyldiethylgermanium, bis-(4-methoxybenzoyl)-diethylgermanium or a mixture thereof, or a mixture of at least one α-diketone and at least one acylgermanium compound.

4. The use according to one of the preceding claims, wherein the material additionally comprises at least one chromophoric component, preferably at least one inorganic pigment based on iron oxide, chromate or molybdate, and/or at least one organic pigment which is selected from azo pigments, such as monoazo, disazo, benzimidazolone and isoindolonone pigments, polycyclic pigments, such as phthalocyanine, thioindigo, flavanthrone, dioxazine and anthanthrone pigments.

5. The use according to one of the preceding claims, wherein the material additionally comprises at least one filler, a phase change agent, a wetting agent and/or a stabilizer.

6. The use according to one of the preceding claims, wherein the material comprises
20 to 98 wt.-%, preferably 40 to 95 wt.-%, particularly preferably 58 to 90 wt.-%, binder (a),
0.5 to 15 wt.-%, preferably 1 to 10 wt.-%, particularly preferably 1.5 to 7 wt.-%, volume expansion component (b),
0.05 to 5 wt.-%, preferably 0.1 to 3 wt.-%, particularly preferably 0.2 to 2 wt.-%, initiator (c), and
0,001 to 5 wt.-%, preferably 0.001 to 2 wt.-%, particularly preferably 0.05 to 1 wt.-% energy transformation component (d),
in each case relative to the total mass of the material.

7. The use according to claim 6, wherein the material additionally comprises
0 to 10 wt.-%, preferably 0 to 8 wt.-%, particularly preferably 0 to 5 wt.-% phase change agent and/or
0 to 78 wt.-%, preferably 0 to 50 wt.-%, particularly preferably 0 to 40 wt.-% filler and/or
0.001 to 0.1 wt.-%, preferably 0.005 to 0.07 wt.-%, particularly preferably 0.01 to 0.05 wt.-% stabilizer and/or
0 to 2 wt.-%, preferably 0 to 1 wt.-%, particularly preferably 0 to 0.5 wt.-% wetting agent,
in each case relative to the total mass of the material.

8. Process for the additive manufacture of three-dimensional objects, which comprises the following steps:
(1) laminar application of a support/construction material to a carrier in a defined layer thickness, preferably in a layer thickness of 3 - 300 µm, particularly preferably 10 - 100 µm,
(2) transfer of a portion of the support/construction material from the carrier substrate (donor) onto a receiver substrate (acceptor) by the local, site-selection input of an energy pulse, preferably a laser pulse,
(3) solidification of the support/construction material on the receiver substrate, preferably by drying, radiation curing or altering the aggregation state,
(4) repetition of steps (1)-(3) until the desired object has been constructed,
(5) optionally removal of the support material and optional cleaning of the object,
(6) optional post-curing of the component by further curing, preferably by drying, radiation, heat or a combination thereof,
(7) optional mechanical processing of the object by e.g. vibratory finishing or manual processing such as grinding or polishing,
**characterized in that** a material as defined in one of claims 1 to 7 is used as the construction material.

9. The process according to claim 8, in which the support/construction material is smoothed following step (3), preferably with a roller, blade, burr and/or a wiper.

10. The process according to claim 8 or 9, in which in step (2) one or more layers of support material are applied to the receiver substrate and then the construction material is deposited/applied onto or into the previously deposited layers of the support material by means of LIFT.

11. The process according to one of claims 8 to 10, in which a polymer film, a glass carrier, a carrier made of a non-metallic, inorganic, non-porous material, a metallic carrier or a ceramic carrier is used as carrier in step (1) and/or receiver substrate in step (2).

12. The process according to claim 11, in which in step (2) a non-porous receiver substrate is used.

13. The process according to one of claims 8 to 12, in which the energy input in step (2) is effected via the side of the carrier substrate facing away from the support or construction material.

## Revendications

1. Utilisation d'un matériau qui contient
(a) au moins un (méth)acrylate mono- ou multifonctionnel ou un mélange de tels composés, en tant que liant polymérisable,
(b) au moins un mono(méth)acrylate en tant que composant d'expansion volumique,
(c) au moins un photoamorceur pour la polymérisation radicalaire et
(d) au moins un colorant organique ou inorganique et/ou au moins un pigment organique ou inorganique en tant que composant de transformation d'énergie,
en tant que matériau de construction pour l'impression par transfert induit par impulsion d'énergie (LIFT).

2. Utilisation selon la revendication 1, dans laquelle le matériau en tant que composant d'expansion volumique (b) contient du (méth)acrylate de méthyle, du (méth)acrylate d'éthyle, du (méth)acrylate de propyle, du (méth)acrylate de butyle, du (méth)acrylate de pentyle, du (méth)acrylate d'hexyle et/ou du N,N-diméthyl-acrylamide.

3. Utilisation selon l'une quelconque des revendications précédentes, dans laquelle le matériau contient en tant que photoamorceur (c)
un photoamorceur pour la région UV, de préférence un oxyde de phosphine, la benzoïne, le benzilcétal, l'acétophénone, la benzophénone, la thioxanthone ou un mélange de ces composés, un oxyde d'acyl- ou bisacylphosphine tel que l'oxyde de 2,4,6-triméthylbenzoyldiphénylphosphine ou l'oxyde de bis-(2,4,6-triméthylbenzoyl)-phénylphosphine, la benzoïne, un éther alkylique de benzoïne, un benzyldialkylcétal tel que le benzyldiméthylcétal, une α-hydroxyacétophénone telle que la 1-hydroxy-cyclohexyl-phénylcétone, la 2-hydroxy-2-méthyl-1-phényl-1-propanone ou la 2-hydroxy-1-[4-(2-hydroxyéthoxy)-phényl]-2-méthyl-1-propanone, une α-dialcoxy-acétophénone, une α-aminoacétophénone telle que la 2-benzyl-2-(diméthylamino)-1-[4-(4-morpholinyl)-phényl]-1-butanone ou la 2-méthyl-1-[4-(méthylthio)-phényl]-2-(4-morpholinyl)-1-propanone, une alkylthioxanthone telle que l'isopropylthioxanthone ou un mélange de celles-ci, un oxyde d'acyl- ou bisacylphosphine, l'oxyde de 2,4,6-triméthylbenzoyldiphénylphosphine, l'oxyde de bis-(2,4,6-triméthylbenzoyl)-phényl-phosphine ou un mélange de ceux-ci,
et/ou
un photoamorceur pour la région visible, de préférence une α-dicétone, un composé acylgermanium, un métallocène ou un mélange de ceux-ci, une α-dicétone telle que la camphoquinone, la 9,10-phénanthrènequinone, la 1-phényl-propane-1,2-dione, le diacétyle, le 4,4'-dichlorobenzile ou un dérivé de celui-ci, un composé monoacyl- ou diacylgermanium, tel que le benzoyltriméthylgermanium, le dibenzoyl-diéthylgermanium ou le bis-(4-méthoxybenzoyl)-diéthylgermanium, un titanocène tel que le bis-(η⁵-2,4-cyclopentadién-1-yl)-bis-[2,6-difluoro-3-(1*H*-pyrrol-1-yl)phényl]-titane ou un mélange de ceux-ci, une α-dicétone telle que la camphoquinone, la 1-phénylpropane-1,2-dione ou un mélange de celles-ci, un composé monoacyltrialkyl-ou diacyldialkylgermanium, le benzoyltriméthylgermanium, le dibenzoyldiéthyl-germanium, le bis-(4-méthoxybenzoyl)diéthylgermanium ou un mélange de ceux-ci, ou un mélange d'au moins une α-dicétone et d'au moins un composé acylgermanium.

4. Utilisation selon l'une quelconque des revendications précédentes, dans laquelle le matériau contient en outre au moins un composant colorant, de préférence au moins un pigment inorganique à base d'oxyde de fer, de chromate ou molybdate, et/ou au moins un pigment organique, qui est choisi parmi les pigments azoïque, tels que les pigments monoazoïques, disazoïques, benzimidazolone et isoindolonone, les pigments polycycliques, tels que les pigments phtalocyanine, thioindigo, flavanthrone, dioxazine et anthantrone.

5. Utilisation selon l'une quelconque des revendications précédentes, dans laquelle le matériau contient en outre au moins une charge, un agent de changement de phase, un agent mouillant et/ou un stabilisant.

6. Utilisation selon l'une quelconque des revendications précédentes, dans laquelle le matériau contient
20 à 98 % en poids, de préférence 40 à 95 % en poids, de façon particulièrement préférée 58 à 90 % en poids de liant (a),
0,5 à 15 % en poids, de préférence 1 à 10 % en poids, de façon particulièrement préférée 1,5 à 7 % en poids de composant d'expansion volumique (b),
0,05 à 5 % en poids, de préférence 0,1 à 3 % en poids, de façon particulièrement préférée 0,2 à 2 % en poids d'amorceur (c) et
0,001 à 5 % en poids, de préférence 0,001 à 2 % en poids, de façon particulièrement préférée 0,05 à 1 % en poids de composant de transformation d'énergie (d),
chaque fois par rapport à la masse totale du matériau.

7. Utilisation selon la revendication 6, dans laquelle le matériau contient en outre
0 à 10 % en poids, de préférence 0 à 8 % en poids, de façon particulièrement préférée 0 à 5 % en poids d'agent de changement de phase et/ou
0 à 78 % en poids, de préférence 0 à 50 % en poids, de façon particulièrement préférée 0 à 40 %p 0 à 40 % en poids de charge et/ou
0,001 à 0,01 % en poids, de préférence 0,005 à 0,07 % en poids, de façon particulièrement préférée 0,01 à 0,05 % en poids de stabilisant et/ou
0 à 2 % en poids, de préférence 0 à 1 % en poids, de façon particulièrement préférée 0 à 0,5 % en poids d'agent mouillant,
chaque fois par rapport à la masse totale du matériau.

8. Procédé pour la fabrication générative d'objets tridimensionnels, qui comprend les étapes suivantes :
(1) application à plat d'une matière de construction/soutien sur un support en épaisseur de couche définie, de préférence en une épaisseur de couche de 3 - 300 µm, de façon particulièrement préférée 10 - 100 µm,
(2) transfert d'une partie de la matière de construction/soutien du substrat porteur (donneur) à un substrat récepteur (accepteur) par l'introduction locale, localement sélective, d'une impulsion d'énergie, de préférence d'une impulsion laser,
(3) consolidation de la matière de construction/soutien sur le substrat récepteur, de préférence par séchage, durcissement par irradiation ou changement de l'état d'agrégation,
(4) répétition des étapes (1) - (3) jusqu'à ce que l'objet souhaité soit construit,
(5) éventuellement élimination de la matière de soutien et en option nettoyage de l'objet,
(6) post-traitement optionnel de la pièce construite, par durcissement plus poussé, de préférence par séchage, irradiation, chaleur ou une combinaison de tels moyens,
(7) finissage mécanique optionnel de l'objet, par exemple par trovalisation ou finissage manuel tel que ponçage ou polissage,
**caractérisé en ce qu'**on utilise comme matière de construction un matériau tel que défini dans l'une quelconque des revendications 1 à 7.

9. Procédé selon la revendication 8, dans lequel à la suite de l'étape (3) on polit la matière de construction/soutien à l'aide d'un rouleau, d'une lame, d'une fraise et/ou d'une racle.

10. Procédé selon la revendication 8 ou 9, dans lequel on applique dans l'étape (2) une ou plusieurs couche(s) de matière de soutien sur le substrat récepteur et on applique/dépose ensuite par LIFT une matière de construction sur ou dans les couches de la matière de soutien déposées précédemment.

11. Procédé selon l'une quelconque des revendications 8 à 10, dans lequel on utilise comme support dans l'étape (1) et/ou substrat récepteur dans l'étape (2) un film en polymère, un support en verre, un support en un matériau non métallique, inorganique, non poreux, un support métallique ou un support céramique.

12. Procédé selon la revendication 11, dans lequel on utilise dans l'étape (2) un substrat récepteur non poreux.

13. Procédé selon l'une quelconque des revendications 8 à 12, dans lequel l'introduction d'énergie dans l'étape (2) s'effectue sur la face du substrat porteur opposée à la matière de construction ou soutien.
